# EUROPEAN PATENT APPLICATION

(11) **EP 4 155 734 A2**
(43) Date of publication of application: **29.03.2023**
(21) Application number: 22193751.9
(22) Date of filing: 07.07.2020
(51) Int. Cl.: G01N 33/569, G01N 33/68, C07K 14/74

(54) **METHOD**

(30) Priority: 08.07.2019 GB 201909774
(62) Divisional of application: 20737275.6
(71) Applicant: Worg Pharmaceuticals (Zhejiang) Co., Ltd., Huzhou, Zhejiang (CN)
(72) Inventor: JANSSON, Liselotte, London (GB); SCHURGERS, Evelien, London (GB); WRAITH, David, London (GB)
(74) Representative: D Young & Co LLP

(57) **Abstract**

The invention relates to methods for identifying tolerogenic peptides. The invention also relates to products used in and produced by such methods. The invention also relates to the use of such tolerogenic peptides in the treatment of disease.

## Description

### FIELD OF THE INVENTION

The invention relates to methods for identifying tolerogenic peptides. The invention also relates to products used in and produced by such methods. The invention also relates to the use of such tolerogenic peptides in the treatment of disease.

### BACKGROUND TO THE INVENTION

In an adaptive immune response, T lymphocytes are capable of recognising internal epitopes of a protein antigen. Antigen presenting cells (APCs) take up protein antigens and degrade them into short peptide fragments. A peptide may bind to a major histocompatibility complex (MHC) class I or II molecule inside the cell and be carried to the cell surface. When presented at the cell surface in conjunction with an MHC molecule, the peptide may be recognised by a T cell (via the T cell receptor (TCR)), in which case the peptide is a T cell epitope.

T cell epitopes play a central role in the adaptive immune response to any antigen, whether self or foreign. The central role played by T cell epitopes in hypersensitivity diseases (which include allergy, autoimmune diseases and transplant rejection) has been demonstrated through the use of experimental models. It is possible to induce immunological or allergic diseases by immunisation with synthetic peptides (based on the structure of T cell epitopes) in combination with adjuvant.

By contrast, it has been shown to be possible to induce immunological tolerance towards particular peptide epitopes by administration of peptide epitopes in soluble form. Administration of soluble peptide antigens has been demonstrated as an effective means of inhibiting disease in experimental autoimmune encephalomyelitis (EAE - a model for multiple sclerosis (MS)) (Metzler and Wraith (1993) Int. Immunol. 5:1159-1165; Liu and Wraith (1995) Int. Immunol. 7:1255-1263; Anderton and Wraith (1998) Eur. J. Immunol. 28:1251 -1261); and experimental models of arthritis, diabetes, and uveoretinitis (reviewed in Anderton and Wraith (1998) as above). This has also been demonstrated as a means of treating an ongoing disease in EAE (Anderton and Wraith (1998) as above, Streeter et al. (2015) Neurology, Neuroimmunology, Neuroinflammation vol. 2, no. 3, e93).

The use of tolerogenic peptides to treat or prevent disease has attracted considerable attention. One reason for this is that it has been shown that certain tolerogenic epitopes can down-regulate responses of T cells for distinct antigens within the same tissue. This phenomenon, known as "bystander suppression" means that it should be possible to induce tolerance to more than one epitope (preferably all epitopes) within a given antigen, and to more than one antigen for a given disease, using a particular tolerogenic peptide (Anderton and Wraith (1998) as above, Wraith (2016) Nature 530: 422-423). This would obviate the need to identify all of the pathogenic antigens within a particular disease.

Peptides are also a favourable option for therapy because of their relatively low cost and the fact that peptide analogues can be produced with altered immunological properties or altered solubility. Peptides may thus be modified to alter their interactions with either MHC or TCR.

One possible problem with this approach is that it has been shown that not all peptides which act as T cell epitopes are capable of inducing tolerance. The myelin basic protein (MBP) peptide 89-101 is an immunodominant antigen after immunisation and is also a very effective immunogen both in terms of priming for T cell reactivity and induction of EAE. However, this peptide has been shown to be ineffective at inducing tolerance when administered in solution (Anderton and Wraith (1998), as above).

The present inventors have previously shown that, if a peptide epitope has appropriate properties to be presented by immature APC without antigen processing, the peptide can induce immunological tolerance. The observation that some T cell epitopes are tolerogenic and others are incapable of inducing tolerance can therefore be explained by the fact that some epitopes require further processing before they are capable of being presented by an MHC molecule. These epitopes which require further processing do not induce tolerance when administered in a soluble form, despite their capacity to induce disease when injected in combination with adjuvant.

The epitopes which do not require further processing are capable of inducing tolerance, and have been termed "apitopes" (Antigen Processing Independent epiTOPES) by the inventors.

This finding provides a rule-based method for selection of tolerogenic T cell epitopes which obviates the need to examine the tolerogenic capacity of a peptide *in vivo,* and is particularly advantageous in the development of strategies to treat or prevent diseases for which no animal models are available.

Even for diseases which have an animal model, the selection method should make the development of tolerance-inducing compositions simpler and safer, because it provides a mechanism whereby the tolerance induction capacity of a peptide can be tested on human T cells (recognising antigen in conjunction with human MHC molecules) *in vitro,* prior to its use *in vivo.*

However, the inventors have now developed an improved method for selecting and/or identifying tolerogenic peptides. The method robustly and rigorously screens for tolerogenic peptides by selecting peptides having multiple properties associated with tolerogenic peptides. Furthermore, the inventors have developed more efficient methods for testing peptides for properties associated with tolerogenicity, and methods which better mimic the natural process of antigenic peptide selection. These improved methods accordingly render the overall process of identifying tolerogenic peptides more efficient and more effective in selecting peptides that will have a tolerogenic effect *in vivo,* and tolerogenic peptides can be identified more rapidly than with methods previously known in the art.

### SUMMARY OF THE INVENTION

The invention relates to several methods (or stages) as described herein that may be carried out in combination, in order to achieve an efficient process for identifying tolerogenic peptides. The methods or stages may encompass any of the following:

### Identification of T cell epitopes - CLIP displacement assay

In one aspect the invention provides a method for identifying a peptide comprising a T cell epitope, wherein said method comprises the steps of:
(i) contacting a complex of major histocompatibility complex class II (MHCII) and Class II-associated invariant chain peptide (CLIP) (MHCII-CLIP) with a peptide;
(ii) adding T cells specific for an antigen; and
(iii) measuring activation of said T cells.

A peptide that leads to T cell activation may comprise a T cell epitope.

The CLIP peptide may have the sequence PVSKMRMATPLLMQA.

In another aspect the invention provides a method for testing the ability of a peptide to bind to MHCII without having undergone processing, wherein said method comprises the steps of:
(i) contacting a complex of major histocompatibility complex class II (MHCII) and Class II-associated invariant chain peptide (CLIP) (MHCII-CLIP) with a peptide;
(ii) adding T cells specific for an antigen; and
(iii) measuring activation of said T cells.

A peptide that leads to T cell activation may be capable of binding to MHCII without having undergone processing.

In another aspect the invention provides use of CLIP in a method for identifying tolerogenic peptides.

In one aspect T cell activation may be measured by measuring the level of secreted IL-2, or any other suitable method. One skilled in the art will be aware of suitable techniques for measuring cytokine levels, for example by ELISA methods or other suitable methods.

### In vitro competition assay

In another aspect the invention provides a method for testing the binding affinity of a peptide for MHCII presented on an APC, wherein said method comprises the steps of:
(i) adding a test peptide to MHCII;
(ii) adding a control peptide;
(ii) adding T cells specific for the control peptide; and
(iii) measuring T cell activation.

In this assay, the control peptide is one which is a known tolerogenic peptide capable of binding to MHCII. The test peptide is added to MHCII/APC in the first instance, and then control peptide is subsequently added. If T cells are activated, the control peptide has displaced the test peptide, and therefore the test peptide has lower relative binding affinity compared to the control peptide. In other words, if the control peptide specific T cell activation is reduced the test peptide binds to MHCII with similar or higher affinity and thereby prevents replacement with the control peptide.

In one aspect the control peptide may be that of SEQ ID NO:10. For example, when the MHCII is HLA-DR2.

In another aspect the invention provides use of a peptide consisting of the sequence KKGPRCLTRYYSSFVNMEGKK (SEQ ID No. 10) in a method for testing the binding affinity of a peptide for MHCII.

In one aspect the control peptide may be that of SEQ ID NO:5. For example, when the MHCII is HLA-DR3 or HLA-DR4.

In another aspect the invention provides use of a peptide consisting of the sequence KKKYVSIDVTLQQLEKKK (SEQ ID No. 5) in a method for testing the binding affinity of a peptide for MHCII.

### In vivo MCHII loading assay

In one aspect, peptides may be modified to improve solubility. Modification of FVIII peptides to improve their solubility is described in WO 2014/072958. Modification of thyroid stimulating hormone receptor (TSHR) peptides to improve their solubility is described in WO 2015/019302. Modification of S-antigen (S-Ag) peptides to improve their solubility is described in WO 2018/127830.

In another aspect, the invention encompasses a method which may determine whether a peptide is sufficiently soluble that it will pass through the fluid phase, and ultimately enter the spleen when injected. In the spleen, the peptide may then bind MHCII on dendritic cells and be presented.

As such, in another aspect the invention provides a method for testing the *in vivo* solubility of a peptide, said method comprising the steps of:
(i) providing dendritic cells from a mouse that has been injected with the peptide;
(ii) co-culturing said dendritic cells with T cells specific for said peptide; and
(iii) measuring T cell activation.

In one aspect the T cells may be primary T cells, T cell clones or T cell hybridomas.

### Ex-vivo tolerance

In another aspect the invention provides an *ex vivo* method for testing the ability of a peptide to induce tolerance to an antigen, said method comprising the steps of:
(i) providing T cells from an animal that has been injected with the peptide;
(ii) stimulating said T cells with the antigen; and
(iii) measuring T cell activation.

In one aspect, T cell activation may be measured by measuring BrdU incorporation, EdU incorporation, Ki67 levels, IL2 secretion, IL17 secretion and/or IFNy secretion.

### Recognition of peptides by patient cells

In one aspect, the CLIP-displacement assay as described herein may be performed, wherein step (ii) comprises adding PBMCs which have been isolated from a patient. Such as assay may be used to confirm that the peptide is presented to patient cells.

### Testing efficacy in disease model

In one aspect the invention may encompass a step of testing the identified peptide in a suitable animal model of the disease in question. One skilled in the art will be aware of suitable disease models. For example, an animal model used to test FVIII peptides is described in WO 2014/072958. An experimental autoimmune encephalomyelitis (EAE) model induced by immunisation with myelin oligodendrocyte glycoprotein (MOG) peptides is described in WO 2014/111840. An animal model of Graves' Disease used for testing thyroid stimulating hormone receptor (TSHR) peptides is described in WO2015/019302.

### Biomarker assay

In another aspect the invention provides a method for determining the effect of a peptide on cytokine secretion, comprising measuring serum cytokine levels of a mouse that has been injected with the peptide. The pattern of cytokines secreted in response to the peptide demonstrates that the peptide binds MHCII and activates T cells *in vivo.* The change in the cytokine secretion pattern after repeated peptide injections, such as increased secretion of IL10, indicates tolerance induction.

In one aspect the mouse may be a HLA-DR transgenic mouse.

In one aspect the invention provides a method for identifying a tolerogenic peptide comprising the steps of:
(i) performing the CLIP-displacement assay according to a method as described herein; and/or
(ii) performing the *in vitro* competition assay according to a method as described herein; and/or
(iii) performing the *in vivo* MCHII loading assay according to a method as described herein; and/or
(iv) performing the CLIP-displacement assay with patient PBMCs according to a method as described herein; and/or
(v) performing the ex vivo tolerance assay according to a method as described herein; and/or
(vi) testing the efficacy of the peptide in a disease model; and/or
(vii) performing the biomarker assay according to a method as described herein.

In another aspect the invention provides a tolerogenic peptide identified by a method of the invention.

In another aspect the invention provides composition comprising a tolerogenic peptide identified by a method of the invention.

In another aspect the invention provides a method for treating and/or preventing a disease in a subject comprising the step of administering a peptide or a composition of the invention to the subject.

In another aspect the invention provides a tolerogenic peptide or a composition for use in treating and/or preventing a disease.

In another aspect the invention provides use of tolerogenic peptide or a composition of the invention in manufacture of a medicament for treatment and/or prevention of a disease.

### DESCRIPTION OF THE FIGURES

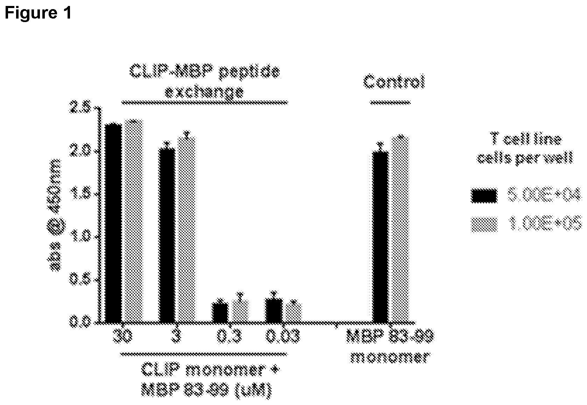

### Figure 1 - CLIP displacement assay

Response of MBP-specific T cell line to HLA-DR2 monomers following peptide exchange. Biotinylated HLA-DR2 (DRB1*1501) monomers, with the CLIP peptide bound, were attached to neutravidin coated 96 well plates. Wells were incubated with the indicated concentration of MBP 83-99, a dominant HLA-DR2 restricted epitope of MBP, for 16 hours and the plates washed. Control wells were coated with the DR2 monomer pre-loaded with the MBP 83-99 peptide. A T cell line specific for MBP was incubated with the monomers at 37°C for 24 hours and response assessed by measurement of secreted IL-2.

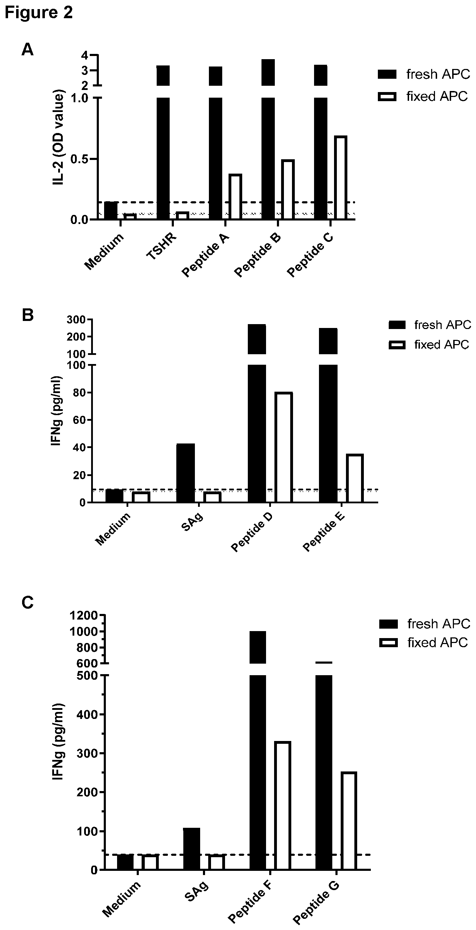

### Figure 2 - APIPS assay

### Response of T cells towards peptide presented by fixed antigen-presenting cells (APCs) to identify apitopes (antigen processing independent epitopes)

**A.** A TSHR-specific hybridoma was co-cultured with fresh or fixed splenocytes from HLA-DR transgenic mice as APCs, in the presence of the indicated protein or peptides. Secretion of IL-2 by responding hybridoma was measured in supernatant of these cultures.
**B.** CD4+ T cells isolated from HLA-DR transgenic mice immunized with peptide D in CFA were co-cultured with fresh or fixed VAVY cells as APCs. Protein or peptide antigen was added to these cultures and IFNg secretion by responding CD4+ cells was measured in supernatant.
**C.** Cells from a peptide F-specific (HLA-DR transgenic) mouse T cell line (TCL) were co-cultured with VAVY cells as APCs. Protein or peptide antigen was added to these cultures and IFNg secretion by responding TCL was measured in supernatant.

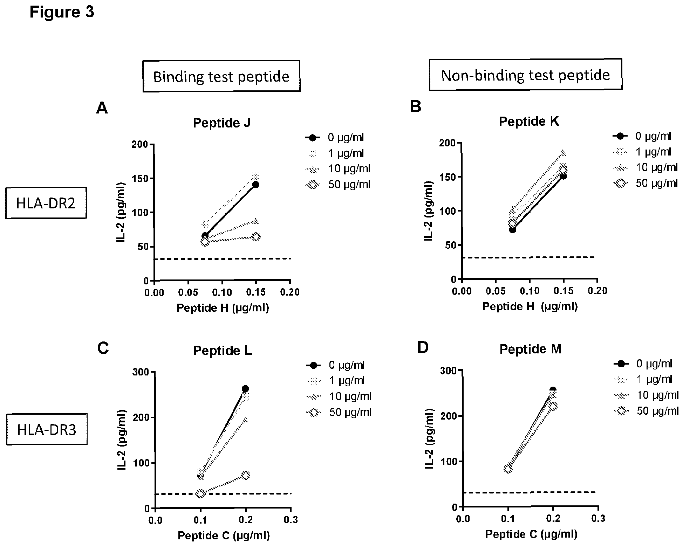

### Figure 3 - In vitro competition assay

Response of control peptide-specific T cell hybridoma's after competition with test peptide. Control peptide-specific T cell hybridoma's were cultured in the presence of fixed HLA-DR-expressing antigen-presenting cells (APCs). Test peptide and control peptide were added to these cultures for 48h at 37°C. The response of the T cell hybridoma's was assessed by measurement of secreted IL-2. Reduced secretion of IL-2 indicate the binding of the test peptide in favour for the control peptide.

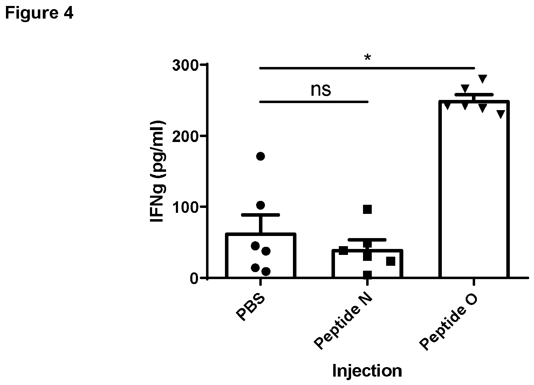

### Figure 4 - In vivo MHCII loading assay

Response of peptide-specific CD4⁺ T cells towards peptide loaded *in vivo* on dendritic cells. HLA-DR-transgenic mice were injected subcutaneously with 100 µg of peptide N (HLA-DR non-binding) or peptide O (HLA-DR binding). 2h post injection, spleens were dissected and CD11c⁺ cells (dendritic cells) were isolated. These CD11c⁺ cells were co-cultured with peptide-specific CD4⁺ cells for 72h at 37°C. IFNy was measured in the supernatant of these cultures to evaluate the response to peptide by the CD4⁺ T cells.

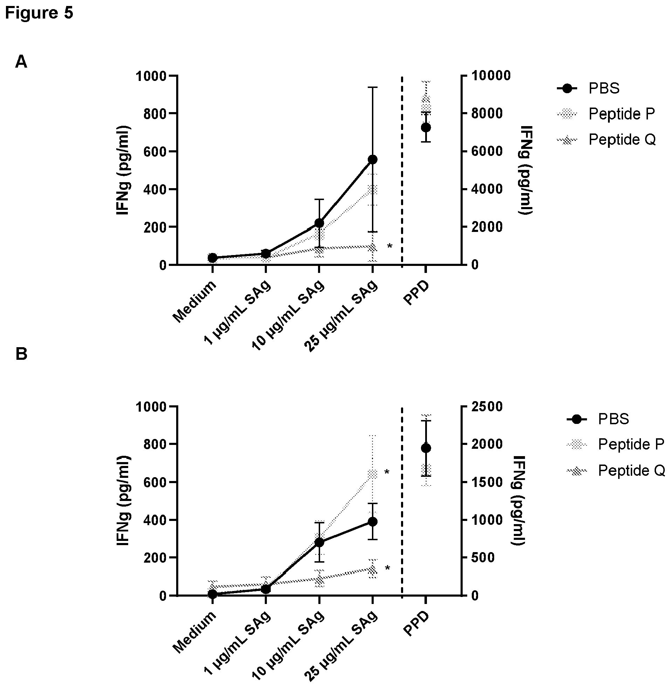

### Figure 5 - Ex vivo tolerance assay

Response to protein antigen in lymph nodes (A) and spleens (B) of peptide-treated and non-treated mice. HLA-DR transgenic mice were treated with a dose-escalating regime of subcutaneous injections (0,1; 1; 10 and 3x 100 µg every other day) of a tolerogenic (peptide Q) or non-tolerogenic (peptide P) peptide, or control injections (PBS). Afterwards, mice were immunized with peptide antigen in CFA and 10 days later, lymph nodes and spleens were dissected. Lymph node and spleen cells were cultured in vitro for 3 days at 37°C in the presence of protein antigen (SAg) or positive control (PPD). The response of the cells towards the protein antigen was evaluated by measuring IFNg levels in supernatant of the cultures. *p<0.05

### Figure 6 - Biomarker assay

HLA-DR transgenic mice were immunized with peptide B in CFA. On day 21 post immunization, 100 µg of peptide C was injected subcutaneously. 2 hours post injection, blood was collected and serum cytokine levels analyzed by an electrochemiluminescent assay (MSD). * p<0.05, ** p<0.01

### Figure 7 - Method for identifying tolerogenic peptides

Flowchart showing a method for identifying tolerogenic peptides. Numbers in brackets refer to where each assay described herein may be used: (1) CLIP displacement assay, (2) competition assay, (3) MHCII loading assay, (4) *ex vivo* tolerance assay, (5) biomarker assay.

### DETAILED DESCRIPTION

The invention relates to methods for selecting or identifying tolerogenic peptides.

As used herein, the term "tolerogenic" means capable of inducing tolerance. "Tolerogenic peptides" are peptides that are likely to be effective in treating hypersensitivity disorders such as autoimmune disease. Tolerogenicity can be tested in animal models and clinical trials.

Certain properties are associated with tolerogenicity. For example, a peptide that is tolerogenic may have one or more of the following properties:
- The peptide comprises a T cell epitope
- The peptide can bind to MHCII without being processed
- The peptide has relatively high affinity for MHCII
- The peptide is sufficiently soluble to be administered and circulate *in vivo,* so as to be able to associate with MHCII on APCs *in vivo*
- The peptide induces tolerance in *ex vivo* assays

Properties such as those listed above may indicate that the peptide may be tolerogenic, or may be important or even necessary for the peptide to be tolerogenic.

The methods of the invention involve testing peptides for properties associated with tolerogenicity. In this way tolerogenic peptides can be identified before they are tested in animal models and human clinical trials.

The methods of the invention identify tolerogenic peptides by testing, analysing or assessing whether a given peptide has multiple properties that are indicative, important or necessary for the peptide to be tolerogenic, such as two or more of the properties listed above, such as three or more, such as four or more, such all five of the properties listed above.

The methods of the invention for identifying tolerogenic peptides may be envisaged as a screening process. Peptides are tested for a particular property associated with tolerogenic peptides. Peptides lacking the tested property are disregarded, whilst peptides having the tested property are retained. The retained peptides are then tested for another property associated with tolerogenic peptides, and the process is repeated, each time testing the retained peptides for a different property and retaining peptides having that property. In this manner, a large suite of potentially tolerogenic peptides may be narrowed to a smaller number of tolerogenic peptides that are highly likely to be effective therapeutically.

The methods of the invention therefore provide robust and rigorous means for identifying tolerogenic peptides. The methods of the invention may reduce the number of "false positives" (i.e. peptides taken on into further testing, such as animal models or clinical trials, but which ultimately prove ineffective therapeutically) identified in comparison to methods previously used to identify tolerogenic peptides thereby saving time, money and other resources.

Thus one aspect of the invention provides a method for identifying a tolerogenic peptide comprising
identifying a peptide comprising a T cell epitope, and/or
testing the ability of the peptide to bind to MHCII without having undergone processing, and/or
testing the binding affinity of the peptide for MHCII, and/or
testing the *in vivo* solubility of the peptide, and/or
testing the ability of a peptide to induce tolerance of an antigen, and/or
testing the effect of the peptide on *in vivo* cytokine secretion.

Such methods may be carried out according to the invention as described herein. In further aspects, the invention relates to improved methods for testing, analysing or assessing a peptide for a property or properties associated with tolerogenic peptides.

One such aspect of the invention provides methods for identifying a peptide comprising a T cell epitope that are more efficient than previous methods. These methods are referred to herein as a "CLIP displacement assay".

Another such aspect of the invention provides methods for testing the binding affinity of a peptide for MHCII that are more physiologically relevant than previous methods. These methods are also referred to herein as a "competition assay".

Another such aspect of the invention provides methods for testing in *vivo* solubility of a peptide, which give results more therapeutically relevant than previous *in vitro* methods. These methods are referred to herein as a "MHCII loading assay".

Another such aspect of the invention provides methods for testing the ability of a peptide to induce tolerance of an antigen. These methods are referred to herein as a "ex *vivo* tolerance assay".

Another such aspect of the invention provides methods for determining the effect of a peptide on cytokine secretion. These methods are referred to herein as a "biomarker assay".

These improved methods for testing properties of peptides may be used as part of the aforementioned method for identifying tolerogenic peptides. In other words, the method of the invention for identifying tolerogenic peptides may comprise one or more of the methods of the invention for testing peptides for properties associated with tolerogenic peptides.

In some embodiments, the method for identifying tolerogenic peptides comprises
a method of the invention for identifying a peptide comprising a T cell epitope, and/or
a method of the invention for testing the capability of the peptide to bind to MHCII without having undergone processing, and/or
a method of the invention for testing the binding affinity of the peptide for MHCII, and/or
a method of the invention for testing the *in vivo* solubility of the peptide, and/or
a method of the invention for testing the ability of a peptide to induce tolerance of an antigen, and/or
a method of the invention for testing the effect of the peptide on *in vivo* cytokine secretion.

### Peptides

The term "peptide" is used in the normal sense to mean a series of residues, typically L-amino acids, connected one to the other, typically by peptide bonds between the α-amino and carboxyl groups of adjacent amino acids. The term includes modified peptides and synthetic peptide analogues.

The peptide of the present invention may be made using chemical methods (Peptide Chemistry, A practical Textbook. Mikos Bodansky, Springer-Verlag, Berlin.). For example, peptides can be synthesized by solid phase techniques (Roberge JY et al. (1995) Science 269: 202-204), cleaved from the resin, and purified by preparative high performance liquid chromatography (e.g., Creighton (1983) Proteins Structures And Molecular Principles, WH Freeman and Co, New York NY). Automated synthesis may be achieved, for example, using the ABI 43 1 A Peptide Synthesizer (Perkin Elmer) in accordance with the instructions provided by the manufacturer.

The peptide may alternatively be made by recombinant means, or by cleavage from a longer polypeptide, which may be followed by modification of one or both ends. The composition of a peptide may be confirmed by amino acid analysis or sequencing (e.g., the Edman degradation procedure).

For practical purposes, there are various other characteristics which the peptide may show. For example, it is important that the peptide is sufficiently stable *in vivo* to be therapeutically useful. The half-life of the peptide *in vivo* may be at least 10 minutes (for example in free form), 15 minutes, 30 minutes, 4 hours, or 24 hours.

The peptide may also demonstrate good bioavailability *in vivo.* The peptide may maintain a conformation *in vivo* which enables it to bind to an MHC molecule at the cell surface without due hindrance.

Sequence identity may be assessed by any convenient method. However, for determining the degree of sequence identity between sequences, computer programs that make multiple alignments of sequences are useful, for instance Clustal W (Thompson et al., (1994) Nucleic Acids Res., 22: 4673-4680). Programs that compare and align pairs of sequences, like ALIGN (Myers et al., (1988) CABIOS, 4: 1-17), FASTA (Pearson et al., (1988) PNAS, 85:2444-2448; Pearson (1990), Methods Enzymol., 183: 63-98) and gapped BLAST (Altschul et al., (1997) Nucleic Acids Res., 25: 3389-3402) are also useful for this purpose. Furthermore, the Dali server at the European Bioinformatics institute offers structure-based alignments of protein sequences (Holm (1993) J. Mol. Biol., 233: 123-38; Holm (1995) Trends Biochem. Sci., 20: 478-480; Holm (1998) Nucleic Acid Res., 26: 316-9).

Multiple sequence alignments and percent identity calculations may be determined using the standard BLAST parameters, (using sequences from all organisms available, matrix Blosum 62, gap costs: existence 11, extension 1).

Alternatively, the following program and parameters may be used: Program: Align Plus 4, version 4.10 (Sci Ed Central Clone Manager Professional Suite). DNA comparison: Global comparison, Standard Linear Scoring matrix, Mismatch penalty = 2, Open gap penalty = 4, Extend gap penalty = 1. Amino acid comparison: Global comparison, BLOSUM 62 Scoring matrix.

Thus included in the scope of the invention are variants of the stated or given sequences, as long as the variant retains the functional activity of the parent i.e. the variants are functionally equivalent, in other words they have or exhibit an activity of the parent peptide as defined herein. Such variants may comprise amino acid substitutions, additions or deletions (including truncations at one or both ends) of the parent sequence e.g. of one or more e.g. 1 to 14 amino acids.

Also included are functionally-equivalent derivatives in which one or more of the amino acids are chemically derivatised, e.g. substituted with a chemical group.

The peptides of the invention may be formulated into a composition as neutral or salt forms. Pharmaceutically acceptable salts include the acid addition salts (formed with free amino groups of the peptide) and which are formed with inorganic acids such as, for example, hydrochloric or phosphoric acids, or such organic acids such as acetic, oxalic, tartaric and maleic acid. Salts formed with the free carboxyl groups may also be derived from inorganic bases such as, for example, sodium, potassium, ammonium, calcium, or ferric hydroxides, and such organic bases as isopropylamine, trimethylamine, 2-ethylamino ethanol, histidine and procaine.

### Antigens

The term "antigen" as used herein refers to an entity that induces an immune response. Inappropriate immune response to an antigen leads to disease. For example, an over-active immune response to an antigen can lead to hypersensitivity disorders such as allergies. In autoimmune diseases an immune response develops against substances that are part of the body, which are therefore termed "self antigens".

The methods of the present invention identify peptides that are able to induce tolerance of antigens (i.e. reduce an immune response to antigens). These tolerogenic peptides may therefore be useful in the treatment of diseases associated with inappropriate immune response to antigens. Each such disease is associated with intolerance of specific antigens, which are different for each disease. For example, the autoimmune disease multiple sclerosis (MS) is known to be associated with intolerance of myelin binding protein (MBP) whereas Graves Disease is associated with intolerance of thyroid stimulating hormone receptor (TSHR).

Treatment of each disease therefore requires identification of peptides that can induce tolerance of the specific antigens inducing an inappropriate immune response in the aetiology of that particular disease.

Accordingly, the methods of the present invention may be used to identify peptides that induce tolerance of any protein antigen. In some embodiments the antigen may be MBP, factor VIII (FVIII), myelin oligodendrocyte glycoprotein (MOG), myelin proteolipid protein (PLP), TSHR, cardiac myosin or S-antigen. Tolerogenic peptides derived from these antigens have been used to demonstrate the efficacy of the methods of the invention (see Figures 1-6).

"Antigen-presenting cells" (APCs) are cells that present antigens on their surface for recognition by cells of the immune system. APCs include dendritic cells, macrophages and B cells. In some embodiments APCs used in the methods of the invention have been isolated from an animal ("primary APCs"). In some embodiments APCs are from APC lines (i.e. immortalised APCs).

### T cells

The term "T cell" as used herein refers to a cell expressing a T cell receptor that is capable of responding to a peptide binding the T cell receptor by proliferating and/or secreting cytokines. In some embodiments T cells are CD4⁺ cells.

In some embodiments the T cells may be primary T cells (T cells isolated from a subject).

In some embodiments the T cells may be T cell clones. T cell clones are T cells with the same antigen-specificity generated by stimulating an isolated T cell to proliferate.

In some embodiments the T cells may be a T cell line. T cell lines are T cells that have been immortalised, such as by transformation with a virus, such as Epstein-Barr virus (EBV).

In some embodiments the T cells may be T cell hybridomas. A T cell hybridoma is a fusion of a primary T cell with an immortal cell, generating an immortal cell that can be activated by T cell receptor signalling.

The term "T cell activation" as used herein refers to the response of a T cell to binding of its T cell receptor by a peptide. This response typically comprises proliferation of the T cell and/or increased secretion of cytokines and/or expression of activation markers. T cell activation may thus be measured, assessed or analysed by measuring proliferation of the T cells and/or measuring levels of cytokines and/or measuring expression of activation markers.

In some embodiments, T cell activation may be assessed by measuring proliferation of the T cells. Techniques for measuring T cell proliferation are known in the art, and include measuring 3H-thymidine, 5-bromo-2'-deoxyuridine (BrdU) or 5-ethynyl-2'-deoxyuridine (EdU) incorporation into replicating DNA of proliferating cells.

In some embodiments, T cell activation may be assessed by measuring cytokine levels. In some embodiments, T cell activation may be assessed by measuring interleukin 2 (IL2) levels. In some embodiments, T cell activation may be assessed by measuring interferon gamma (IFNγ) levels.

T cells may be described herein as being specific for particular epitopes, peptides or antigens, or may be described as "peptide-specific" or "antigen-specific". These terms mean that the T cell is activated (i.e. proliferates and/or increases cytokine production and/or increases expression of activation markers) in response to the entity in question. The T cell may bear a T cell receptor that binds to the entity in question, or part of the entity in question, as part of activating the T cell. For example, a "peptide-specific T cell" may be a T cell bearing a T cell receptor capable of associating with a particular peptide bound to MHCII on an APC. A T cell specific for a particular antigen may bear a T cell receptor capable of associating with a peptide derived from that antigen by antigen processing and presented bound to MHCII on an APC.

### Apitopes

In an adaptive immune response, T lymphocytes are capable of recognising epitopes of a protein antigen. Antigen presenting cells (APCs) take up protein antigens and degrade them into short peptide fragments. A peptide may bind to a major histocompatibility complex (MHC) inside the cell and be carried to the cell surface. When presented at the cell surface in conjunction with an MHC molecule, the peptide may be recognised by a T cell via the T cell receptor (TCR), in which case the peptide is a T cell epitope.

An epitope is thus a peptide derivable from an antigen which is capable of binding to the peptide binding groove of an MHC molecule and being recognised by a T cell.

The present inventors have previously determined that there is a link between the capacity of a peptide to bind to an MHC molecule and be presented to a T cell without further processing, and the peptide's capacity to induce tolerance *in vivo* (WO 02/16410). If a peptide is too long to bind the peptide binding groove of an MHC molecule without further processing (e.g. trimming), or binds in an inappropriate conformation then it will not be tolerogenic *in vivo.* If, on the other hand, the peptide is of an appropriate size and conformation to bind directly to the MHC peptide binding groove in the correct formation to be presented to a T cell, then this peptide can be predicted to be useful for tolerance induction.

It is thus possible to investigate the tolerogenic capacity of a peptide by investigating whether it can bind to an MHC molecule and be presented to a T cell without further antigen processing *in vitro.*

Epitopes which do not require further processing are capable of inducing tolerance, and have been termed apitopes (Antigen Processing-Independent epiTOPES) by the inventors.

Peptides which bind to MHC class II molecules are typically between 8 and 20 amino acids in length, more usually between 10 and 17 amino acids in length, and can be longer (for example up to 40 amino acids). These peptides lie in an extended conformation along the MHC II peptide binding groove which (unlike the MHC class I peptide binding groove) is open at both ends. The peptide is held in place mainly by main-chain atom contacts with conserved residues that line the peptide binding groove.

### Tolerance

T cell epitopes play a central role in the adaptive immune response to any antigen, whether self or foreign. The central role played by T cell epitopes in hypersensitivity (autoimmune) diseases (which include allergy and transplant rejection) has been demonstrated through the use of experimental models. It is possible to induce autoimmune or allergic diseases by injection of synthetic peptides (based on the structure of T cell epitopes) in combination with adjuvant.

By contrast, it has been shown to be possible to induce immunogenic tolerance towards particular antigens by administration of peptide epitopes in soluble form. Administration of soluble peptide has been demonstrated as an effective means of inhibiting disease in experimental autoimmune encephalomyelitis (EAE - a model for multiple sclerosis (MS)) (Metzler and Wraith (1993) Int. Immunol. 5:1159-1165; Liu and Wraith (1995) Int. Immunol. 7:1255-1263; Anderton and Wraith (1998) Eur. J. Immunol. 28:1251-1261; de Souza (2018) Neuro. Ther. 7:103-128); and experimental models of arthritis, diabetes, and uveoretinitis (reviewed in Anderton and Wraith (1998) as above). This has also been demonstrated as a means of treating an ongoing disease in EAE (Anderton and Wraith (1998) as above).

Tolerance is the failure to respond to an antigen. Tolerance to self antigens is an essential feature of the immune system. When tolerance of self antigens is lost, autoimmune disease can result. The adaptive immune system must maintain the capacity to respond to an enormous variety of infectious agents while avoiding autoimmune attack of the self antigens contained within its own tissues. This is controlled to a large extent by negative selection of high-affinity T lymphocytes in the thymus (central tolerance). However, not all self antigens are expressed in the thymus, so death of self-reactive thymocytes remains incomplete. There are thus also mechanisms by which tolerance may be acquired by mature self-reactive T lymphocytes in the peripheral tissues (peripheral tolerance). A review of the mechanisms of central and peripheral tolerance is given in Anderton et al. (1999) Immunological Reviews 169:123-137. See also Wraith (2016) Nature 530:422-423.

Tolerance may result from or be characterised by the induction of anergy in at least a portion of CD4+ T cells. In order to activate a T cell, a peptide must associate with a "professional" APC capable of delivering two signals to T cells. The first signal (signal 1) is delivered by the MHC-peptide complex on the cell surface of the APC and is received by the T cell via the TCR. The second signal (signal 2) is delivered by costimulatory molecules on the surface of the APC, such as CD80 and CD86, and received by CD28 on the surface of the T cell. It is thought that when a T cell receives signal 1 in the absence of signal 2, it is not activated and, in fact, becomes anergic. Anergic T cells are refractory to subsequent antigenic challenge, and may be capable of suppressing other immune responses. Anergic T cells are thought to be involved in mediating T cell tolerance.

Peptides which require processing before they can be presented in conjunction with MHC molecules do not induce tolerance because they have to be handled by mature antigen presenting cells. Mature antigen presenting cells (such as macrophages, B cells and dendritic cells) are capable of antigen processing, but also of delivering both signals 1 and 2 to a T cell, leading to T cell activation. Apitopes, on the other hand, will be able to bind directly to MHC class II on immature APC. Thus they will be presented to T cells without costimulation, leading to T cell anergy and tolerance.

Of course, apitopes are also capable of binding to MHC molecules at the cell surface of mature APC. However, the immune system contains a greater abundance of immature than mature APC (it has been suggested that less than 10% of dendritic cells are activated, Summers et al. (2001) Am. J. Pathol. 159: 285-295). The default position to an apitope will therefore be anergy/tolerance, rather than activation.

It has been shown that, when tolerance is induced, the capacity of antigen-specific CD4⁺ T cells to proliferate is reduced. Also, the production of IL2, IFNy and IL4 production by these cells is down-regulated, but production of IL10 is increased. Neutralisation of IL10 in mice in a state of peptide-induced tolerance has been shown to restore completely susceptibility to disease. It has been proposed that a population of regulatory cells persist in the tolerant state which produce IL10 and mediate immune regulation (Burkhart et al. (1999) Int. Immunol. 11: 1625-1634).

The induction of tolerance can therefore be monitored by various techniques including:
(a) reduced susceptibility to contract the disease for which the peptide is a target epitope *in vivo*;
(b) the induction of anergy in CD4⁺ T cells (which can be detected by subsequent challenge with antigen *in vitro*);
(c) changes in the CD4+ T cell population, including
   (i) reduction in proliferation;
   (ii) down-regulation in the production of, for example, IL2, IFNy and IL4; and
   (iii) increase in the production of IL10.

As used herein, the term "tolerogenic" means capable of inducing tolerance.

The methods of the invention aim to identify tolerogenic peptides. In particular, the methods of the invention may be used to identify peptides that are good candidates for further testing in animal models and clinical trials; the methods of the invention provide a strong indication that the peptides are likely to be therapeutically effective. The term "tolerogenic peptides" can therefore refer to peptides that are candidates for further testing in animal models and clinical trials and are likely to be effective in treating autoimmune disease.

### Identifying peptides comprising T cell epitopes

There are a number of methods known in the art to identify T cell epitopes within a given antigen.

Naturally processed epitopes may be identified by mass spectrophotometric analysis of peptides eluted from antigen-loaded APC. These are APC that have either been encouraged to take up antigen, or have been forced to produce the protein intracellularly by transformation with the appropriate gene. Typically APC are incubated with protein either in solution or suitably targeted to the APC cell surface. After incubation at 37°C the cells are lysed in detergent and the class II protein purified by, for example affinity chromatography. Treatment of the purified MHC with a suitable chemical medium (for example, acid conditions) results in the elution of peptides from the MHC. This pool of peptides is separated and the profile compared with peptide from control APC treated in the same way. The peaks unique to the protein expressing/fed cells are analysed (for example by mass spectrometry) and the peptide fragments identified. This procedure usually generates information about the range of peptides (usually found in "nested sets") generated from a particular antigen by antigen processing.

Another method for identifying epitopes is to screen a synthetic library of peptides which overlap and span the length of the antigen in an *in vitro* assay. For example, peptides which are 15 amino acids in length and which overlap by 5 or 10 amino acids may be used. The peptides are tested in an antigen presentation system which comprises antigen presenting cells and T cells. For example, the antigen presentation system may be a murine splenocyte preparation, a preparation of human cells from tonsil or PBMC. Alternatively, the antigen presentation system may comprise a particular T cell line/clone and/or a particular antigen presenting cell type.

T cell activation may be measured via T cell proliferation (for example using ³H-thymidine incorporation) or cytokine production. Activation of TH1-type CD4+ T cells can, for example be detected via IFNy production which may be detected by standard techniques, such as an ELISPOT assay.

Overlapping peptide studies usually indicate the area of the antigen in which an epitope is located. The minimal epitope for a particular T cell can then be assessed by measuring the response to truncated peptides. For example if a response is obtained to the peptide comprising residues 1-15 in the overlapping library, sets which are truncated at both ends (i.e. 1-14, 1-13, 1-12 etc. and 2-15, 3-15, 4-15 etc.) can be used to identify the minimal epitope.

The present inventors have developed a faster method for identifying peptides comprising T cell epitopes. This method is referred to herein as the CLIP displacement assay.

### CLIP displacement assay

Major histocompatibility complexes (MHCs) are multi-sub-unit, cell surface protein complexes that bind antigens and display them on the surface of antigen presenting cells for recognition by T cells. MHCs are divided into three groups: MHC class I, class II and class III. MHC class II (MHCII) is typically expressed on the surface of macrophages, dendritic cells and B cells.

Class II-associated invariant chain peptide (CLIP) is a peptide capable of binding to the peptide binding groove of MHCII that assists in formation and transport of MHCII in antigen presenting cells.

CLIP is synthesised as part of a polypeptide called HLA-DR antigens-associated invariant chain ("invariant chain" for short). In the endoplasmic reticulum (ER), a segment of the invariant chain binds to the peptide binding groove of newly synthesised MHCII complexes that are not yet fully assembled. Invariant chain thereby blocks binding of peptides found in the ER to the peptide binding groove of MHCII. Additionally, a signal in the N-terminal cytoplasmic tail of the invariant chain facilitates export of the invariant chain-MHCII complex from the ER to an endosomal compartment, rather than to the cell surface. In the endosomal compartment, cathepsinS cleaves the invariant chain leaving a fragment (amino acids 86-100), termed CLIP, bound in the peptide binding groove of the MHCII complex. The rest of the invariant chain is degraded.

Within the endosomes, exogenous proteins (i.e. potential antigens) are unfolded and degraded by various proteases (i.e. they undergo "antigen processing"). Peptides from degraded exogenous proteins that are able to displace CLIP from MHCII are selected for antigen presentation.

The inventors have developed a method for identifying a peptide comprising a T cell epitope based on the concept of antigen peptides being able to displace CLIP from the MHCII peptide binding groove. If a peptide can displace CLIP bound to MHCII and activate T cells, that peptide would appear to comprise a T cell epitope.

In particular, the CLIP displacement assay may be used to identify T cell epitopes in an antigen. Wells containing MHCII-CLIP monomers are incubated with a range of peptides covering regions of the antigen predicted to bind to MHCII (i.e. regions predicted to comprise a T cell epitope). Peptides able to bind to MHCII without antigen processing and with sufficient affinity to displace CLIP will be presented. T cells specific for the antigen are used to screen for presentation of peptides.

The CLIP displacement assay thus mimics and reproduces the natural process of antigen-derived peptides binding to MHCII. Hence the peptides selected by the CLIP displacement assay mimic naturally processed T cell epitopes and therefore engage relevant cells for tolerance induction.

The CLIP displacement assay also provides a high throughput method for identification of T cell epitopes, as many different peptides may be tested simultaneously with very little manipulation. For example, in a 96-well plate with wells coated in MHCII-CLIP, 48 duplicate samples may be tested by straight-forwardly adding peptide then T cells to each well.

Thus in one aspect the invention provides method for identifying a peptide comprising a T cell epitope comprising adding a peptide to MHCII having bound Class II-associated invariant chain peptide (CLIP) (MHCII-CLIP), adding T cells specific for an antigen, and measuring T cell activation.

If the peptide being tested comprises a T cell epitope, the peptide may bind to MHCII and displace CLIP. T cells specific for the antigen will then bind to the peptide via their T cell receptors and be activated. Therefore T cell activation indicates that the tested peptide comprises a T cell epitope.

In some embodiments the assay comprises:
(i) selecting T cells responding to the intact antigen (i.e. responding to naturally processed epitopes of the antigen;
(ii) preparing MHCII-CLIP complexes and adhering these to wells of a tissue culture plate;
(iii) incubating the wells with a range of peptides covering a predicted MHCII binding region; and
(iv) adding the T cells and measuring T cell activation.

Furthermore, if a peptide can displace CLIP bound to MHCII and activate T cells, that peptide is capable of binding to MHCII without needing to undergo processing. Only those peptides able to bind without antigen processing and with sufficient affinity to displace CLIP will be presented, thereby providing a high throughput screen for apitopes.

Thus in another aspect the invention provides a method for testing the ability of the peptide to bind to MHCII without having undergone processing, the method comprising adding a peptide to MHCII-CLIP, adding T cells specific for an antigen, and measuring T cell activation.

In some embodiments the peptide is derived from the antigen (i.e. the peptide has the same amino acid sequence as a portion of the antigen). In some embodiments the peptide is derived from a region of the antigen predicted to bind MHCII. In some embodiments the peptide is derived from a region of the antigen predicted to bind contain a T cell epitope.

In some embodiments the peptide is one of a plurality of peptides designed to cover a region in an antigen predicted to contain a T cell epitope or predicted to bind to MHCII. The predictions may have been carried out *in silico.*

In some embodiments the method is carried out multiple times in parallel, in each case using a different peptide, such as different peptides from a plurality of peptides designed to cover a region in an antigen predicted to contain a T cell epitope or predicted to bind to MHCII. Accordingly, peptides comprising a T cell epitope and capable of binding to MHCII without undergoing antigen processing are selected from the plurality of peptides.

In some embodiments the T cells are primary T cells. In some embodiments the T cells are T cell clones. In some embodiments the T cells are T cell hybridomas. In some embodiments the T cells are from a T cell line. In some embodiments the T cells are from peripheral blood mononuclear cells (PBMCs) isolated from a subject.

In some embodiments MHCII may be Human Leukocyte Antigen - DR isotype 2 (HLA-DR2). In some embodiments MHCII may be Human Leukocyte Antigen - DR isotype 2 (HLA-DR3).

In some embodiments T cell activation may be measured by measuring cytokine secretion. In some embodiments T cell activation may be measured by measuring IL2 secretion. In some embodiments T cell activation may be measured by measuring IFNy secretion. In some embodiments T cell activation may be measured by measuring both IL2 secretion and IFNy secretion.

In some embodiments T cell activation may be measured by measuring T cell proliferation. In some embodiments T cell activation may be measured by measuring ³H-thymidine incorporation. In some embodiments T cell activation may be measured by measuring BrdU incorporation. In some embodiments T cell activation may be measured by measuring EdU incorporation. In some embodiments T cell activation may be measured by measuring Ki67 levels.

In some embodiments T cell activation may be measured by measuring both cytokine secretion and T cell proliferation.

In some embodiments CLIP comprises the sequence PVSKMRMATPLLMQA.

In some embodiments the method is an *in vitro* method.

The components of MHCII may be produced as recombinant proteins. In particular, the MHCII alpha and beta chains may be expressed in bacteria, such as *E*. *coli,* then folded and assembled into the MHCII complex with tagged peptides. Methods are known in the art for expressing recombinant proteins, for purifying such proteins, and for assembling proteins into a complex. Day et al. (2003) J. Clin. Investig. 112: 831-842 describes a method for expression of MHCII-CLIP.

The MHCII-CLIP complex may be assembled then affixed to a culture plate, such as wells of a culture plate.

Thus in some embodiments MHCII-CLIP is affixed to a culture plate. The MHCII-CLIP complex may be biotinylated to enable it to be affixed to the culture plate by coating the plate with neutravidin before adding the biotin-MHCII-CLIP complex. In some embodiments MHCII-CLIP comprises biotin. In some embodiments MHCII-CLIP is affixed to a culture plate by biotin-neutravidin.

In another aspect the invention provides use of CLIP in a method of the invention for identifying a peptide comprising a T cell epitope or for testing the ability of the peptide to bind to MHCII without having undergone processing. In some embodiments CLIP comprises the sequence PVSKMRMATPLLMQA (SEQ ID No. 1).

The CLIP displacement assay identifies peptides comprising T cell epitopes and tests the ability of peptides to bind MHCII without having undergone processing. As described herein, the properties of comprising a T cell epitope and being able to being MHCII without processing are associated with tolerogenic peptides. The CLIP displacement assay may thus be used in a method for identifying tolerogenic peptides.

Accordingly, in another aspect the invention provides a method for identifying a tolerogenic peptide comprising adding a peptide to MHCII having bound CLIP, adding T cells specific for an antigen, and measuring T cell activation.

In some embodiments the CLIP displacement assay may be combined with one or more of the other methods described herein for testing peptides for properties associated with tolerogenicity. Thus in some embodiments the invention provides a method for identifying a tolerogenic peptide comprising adding a peptide to MHCII-CLIP, adding T cells specific for an antigen, and measuring T cell activation, wherein the method further comprises a method for identifying a peptide comprising a T cell epitope, and/or a method for testing the ability of a peptide to bind to MHCII without having undergone processing, and/or a method for testing the binding affinity of the peptide for MHCII, and/or a method for testing *ex vivo* the *in vivo* solubility of the peptide, and/or an *ex vivo* method for testing the ability of the peptide to induce tolerance of an antigen, and/or a method for determining the effect of the peptide on cytokine secretion.

In some embodiments the CLIP displacement assay is used to identify human T cell epitopes. The T cells used in the assay may be T cells from a subject suffering from a hypersensitivity disease, such as an autoimmune disease. The T cells would therefore be specific for an antigen to which the subject is hypersensitive. The peptides assessed using the assay may be designed to cover regions of the antigen to which the subject is hypersensitive.

In some embodiments the CLIP displacement assay is used in parallel with identification of T cell epitopes in HLA-DR transgenic mice.

### Antigen Processing Independent Presentation Systems (APIPS)

When identifying tolerogenic peptides, once an epitope has been identified the next step is to investigate whether it also behaves as an apitope (i.e. is capable of binding to MHCII without undergoing processing).

An apitope must be presented to T cells without the need for antigen processing. Having identified peptides containing T cell epitopes, apitopes may be identified using a processing free system.

Peptides may be tested for the ability to bind MHCII without undergoing processing using the CLIP displacement assay of the invention described herein. Alternatively or additionally, the ability to bind MHCII without undergoing processing may be tested using an antigen processing independent presentation system (APIPS).

Examples of APIPS include:
a) fixed APC (with or without antibodies to CD28);
b) lipid membranes containing Class I or II MHC molecules (with or without antibodies to CD28); and
c) purified natural or recombinant MHC in plate-bound form (with or without antibodies to CD28).

It is known to use fixed APC to investigate T cell responses, for example in studies to investigate the minimal epitope within a polypeptide, by measuring the response to truncated peptides (Fairchild et al. (1996) Int. Immunol. 8:1035-1043). APC may be fixed using, for example formaldehyde (usually paraformaldehyde) or glutaraldehyde.

Lipid membranes (which may be planar membranes or liposomes) may be prepared using artificial lipids or may be plasma membrane/microsomal fractions from APC.

In use, the APIPS may be applied to the wells of a tissue culture plate. Peptide antigens are then added and binding of the peptide to the MHC portion of the APIPS is detected by addition of selected T cells, such as primary T cells, T cell hybridomas, T cell lines or T cell clones. Activation of the T cells may be measured by any of the methods known in the art, for example via ³H-thymidine incorporation or cytokine secretion.

Accordingly, in some embodiments the methods of the invention comprise testing the ability of the peptide to bind to MHCII without having undergone processing by treating an APIPS with the peptide, adding T cells specific for the peptide to the APIPS, and measuring T cell activation.

In some embodiments the APIPS comprises fixed APCs. In some embodiments the APCs are primary APCs, such as splenocytes. In some embodiments the APCs are from an APC line, such as MGAR cells or VAVY cells.

In some embodiments the T cells added to the APIPS are primary T cells. In some embodiments the T cells are T cell clones. In some embodiments the T cells are T cell hybridomas. In some embodiments the T cells are from a T cell line.

In some embodiments MHCII may be Human Leukocyte Antigen - DR isotype 2 (HLA-DR2). In some embodiments MHCII may be Human Leukocyte Antigen - DR isotype 2 (HLA-DR3).

In some embodiments the APCs are splenocyte and the T cells are T cell hybridomas. In some embodiments the APCs are from an APC line and the T cells are primary T cells.

In some embodiments T cell activation may be measured by measuring cytokine secretion. In some embodiments T cell activation may be measured by measuring IL2 secretion. In some embodiments T cell activation may be measured by measuring IFNy secretion. In some embodiments T cell activation may be measured by measuring both IL2 secretion and IFNy secretion.

In some embodiments T cell activation may be measured by measuring T cell proliferation. In some embodiments T cell activation may be measured by measuring BrdU incorporation. In some embodiments T cell activation may be measured by measuring EdU incorporation. In some embodiments T cell activation may be measured by measuring Ki67 levels.

In some embodiments T cell activation may be measured by measuring both cytokine secretion and T cell proliferation.

In the methods of the invention for identifying a tolerogenic peptide, an APIPS assay may be used instead of or in addition to the CLIP displacement assay described herein for testing the ability of a peptide to bind to MHCII without having undergone processing. Use of both a CLIP displacement assay and an APIPS assay provides particularly robust identification of peptides able to bind to MHCII without having undergone processing.

Thus, in some embodiments the invention provides a method for identifying a tolerogenic peptide comprising a CLIP displacement assay as described herein and an APIPS assay as described herein. In some embodiments the invention provides a method for identifying a tolerogenic peptide comprising adding a peptide to MHCII-CLIP, adding T cells specific for the peptide, and measuring T cell activation, and then treating an APIPS with the peptide, adding T cells specific for the peptide to the APIPS, and measuring T cell activation.

Furthermore, in some embodiments an APIPS assay may be combined with one or more of the other methods described herein for testing peptides for properties associated with tolerogenicity. Thus in some embodiments the invention provides a method for testing the ability of a peptide to bind to MHCII without having undergone processing comprising treating an APIPS with the peptide, adding T cells specific for the peptide to the APIPS, and measuring T cell activation, wherein the method further comprises a method for identifying a peptide comprising a T cell epitope, and/or a method for testing the ability of a peptide to bind to MHCII without having undergone processing, and/or a method for testing the binding affinity of the peptide for MHCII, and/or a method for testing the *in vivo* solubility of the peptide, and/or an *ex vivo* method for testing the ability of the peptide to induce tolerance of an antigen, and/or a method for determining the effect of the peptide on cytokine secretion.

In some embodiments, the APIPS assay is carried out after the CLIP displacement assay.

### Testing affinity - in vitro competition assay

To be tolerogenic a peptide should have relatively high affinity for MHCII. Binding affinity of molecules can be measured using techniques known in the art. However, the present inventors have established a functional "competition assay" for analysing binding of a peptide to MHCII tailored to the requirements of identifying tolerogenic peptides.

In the competition assay of the invention, a test peptide and a control peptide compete for binding to MHCII. A test peptide that outcompetes the control peptide for binding to MHCII has a relatively high affinity for MHCII so is likely to be a tolerogenic peptide.

Thus in another aspect the invention provides a method for testing the binding affinity of a peptide for MHCII comprising adding a test peptide and a control peptide to MHCII, adding T cells specific for the control peptide, and measuring T cell activation.

In some embodiments MHCII is on antigen presenting cells (APCs). In some embodiments the APCs are fixed. In some embodiments the APCs are "fresh" i.e. not fixed. In some embodiments the APCs are primary APCs. In some embodiments the APCs are from an APC line. In some embodiments the APCs are MGAR cells. In some embodiments the APCs are VAVY cells. In some embodiments the APCs are BM14 cells.

In some embodiments MHCII is HLA-DR2. In some embodiments the control peptide consists of the sequence KKGPRCLTRYYSSFVNMEGKK (SEQ ID No. 10). This control peptide sequence is specific for HLA-DR2.

In some embodiments MHCII is HLA-DR3. In some embodiments MHCII is HLA-DR4. In some embodiments MHCII the control peptide consists of the sequence KKKYVSIDVTLQQLEKKK (SEQ ID No. 5). This control peptide sequence is specific for HLA-DR3 and HLA-DR4.

In some embodiments the T cells are primary T cells. In some embodiments the T cells are T cell clones. In some embodiments the T cells are T cell hybridomas. In some embodiments the T cells are from a T cell line.

In some embodiments T cell activation may be measured by measuring cytokine secretion. In some embodiments T cell activation may be measured by measuring IL2 secretion. In some embodiments T cell activation may be measured by measuring IFNy secretion. In some embodiments T cell activation may be measured by measuring both IL2 secretion and IFNy secretion.

In some embodiments T cell activation may be measured by measuring T cell proliferation. In some embodiments T cell activation may be measured by measuring ³H-thymidine incorporation. In some embodiments T cell activation may be measured by measuring BrdU incorporation. In some embodiments T cell activation may be measured by measuring EdU incorporation. In some embodiments T cell activation may be measured by measuring Ki67 levels.

In some embodiments T cell activation may be measured by measuring both cytokine secretion and T cell proliferation.

In some embodiments the test peptide is added to MHCII before the control peptide. In some embodiments the test peptide is incubated with MHCII for about 15 to 45 minutes before adding the control peptide.

In some embodiments the method is an *in vitro* method.

In another aspect the invention provides use of a control peptide consisting of the sequence KKGPRCLTRYYSSFVNMEGKK (SEQ ID No. 10) in a method of the invention.

In another aspect the invention provides use of a control peptide consisting of the sequence KKKYVSIDVTLQQLEKKK (SEQ ID No. 5) in a method of the invention.

In some embodiments the competition assay of the invention may be combined with one or more of the other methods described herein for testing peptides for properties associated with tolerogenicity. Thus in some embodiments the invention provides a method for identifying a tolerogenic peptide comprising the competition assay of the invention. In some embodiments the invention provides a method for identifying a tolerogenic peptide comprising adding a test peptide and a control peptide to MHCII, adding T cells specific for the control peptide, and measuring T cell activation, wherein the method further comprises a method for identifying a peptide comprising a T cell epitope, and/or a method for testing the ability of a peptide to bind to MHCII without having undergone processing, and/or a method for testing the *in vivo* solubility of the peptide, and/or an *ex vivo* method for testing the ability of the peptide to induce tolerance of an antigen, and/or a method for determining the effect of the peptide on cytokine secretion.

In some embodiments, the competition assay of the invention is carried out on a peptide identified as comprising a T cell epitope, such as by a CLIP displacement assay of the invention. In some embodiments, the competition assay of the invention is carried out on a peptide identified as being able to bind MCHII without having undergone processing, such as by a CLIP displacement assay of the invention or by an APIPS assay. In some embodiments, a peptide is tested first by a CLIP displacement assay of the invention, then by an APIPS assay, then by the competition assay of the invention.

In some embodiments the competition assay of the invention is combined with an APIPS assay. In other words, the APIPS assay and competition assay are carried out simultaneously.

### Testing solubility - MHCII loading assay

Dendritic cells are a type of antigen presenting cell that expresses MHCII found in lymphoid organs (lymph nodes and spleen). Peptides that can bind MHCII on splenic dendritic cells *in vivo* without undergoing antigen processing can induce tolerance. However, to reach the dendritic cells in the lymphoid organs the peptides must be sufficiently soluble to pass through the fluid phase. The degree to which peptides are able to bind MHCII on splenic dendritic cells may be termed "MHCII loading".

The present inventors have identified a direct correlation between the ability of a peptide to bind MHCII on dendritic cells *in vivo* and its ability to induce tolerance. When identifying tolerogenic peptides potentially useful in the treatment of disease, it is therefore useful to know whether the peptides can bind to MHCII on dendritic cells *in vivo.* The inventors have developed an assay to test peptides for this property, referred to herein as the MHCII loading assay.

Thus in another aspect the invention provides a method for testing the *in vivo* solubility of a peptide comprising co-culturing dendritic cells from a mouse that has been injected with the peptide with T cells specific for the peptide, and measuring T cell activation, wherein the T cells are primary T cells, T cell clones or T cell hybridomas.

The term "dendritic cells" may refer to CD11c⁺ cells. Such cells may be isolated using techniques known in the art.

In some embodiments the T cells are primary T cells. In some embodiments the T cells are T cell clones. In some embodiments the T cells are T cell hybridomas.

In some embodiments T cell activation may be measured by measuring cytokine secretion. In some embodiments T cell activation may be measured by measuring IL2 secretion. In some embodiments T cell activation may be measured by measuring IFNy secretion. In some embodiments T cell activation may be measured by measuring both IL2 secretion and IFNy secretion.

In some embodiments T cell activation may be measured by measuring T cell proliferation. In some embodiments T cell activation may be measured by measuring ³H-thymidine incorporation. In some embodiments T cell activation may be measured by measuring BrdU incorporation. In some embodiments T cell activation may be measured by measuring EdU incorporation. In some embodiments T cell activation may be measured by measuring Ki67 levels.

In some embodiments T cell activation may be measured by measuring both cytokine secretion and T cell proliferation.

In some embodiments the mouse is a HLA-DR2 transgenic (HLA-DR2tg) or HLA-DR3 transgenic (HLA-DR3tg) mouse.

In some embodiments the mouse has been injected with about 100 µg of peptide. In some embodiments the mouse has been injected subcutaneously.

In some embodiments the dendritic cells are harvested from the spleen of the mouse.

In some embodiments the MHCII loading assay of the invention may be combined with one or more of the other methods described herein for testing peptides for properties associated with tolerogenicity. Thus in some embodiments the invention provides a method for identifying a tolerogenic peptide comprising the MHCII loading assay of the invention. In some embodiments the invention provides a method for identifying a tolerogenic peptide comprising co-culturing dendritic cells from a mouse that has been injected with the peptide with T cells specific for the peptide, and measuring T cell activation, wherein the T cells are primary T cells, T cell clones or T cell hybridomas, wherein the method further comprises a method for identifying a peptide comprising a T cell epitope, and/or a method for testing the ability of a peptide to bind to MHCII without having undergone processing, and/or a method for testing the binding affinity of the peptide for MHCII, and/or an *ex vivo* method for testing the ability of the peptide to induce tolerance of an antigen, and/or a method for determining the effect of the peptide on cytokine secretion.

In some embodiments, the MHCII loading assay of the invention is carried out on a peptide identified as comprising a T cell epitope, such as by a CLIP displacement assay of the invention. In some embodiments, the MHCII loading assay of the invention is carried out on a peptide identified as being able to bind MCHII without having undergone processing, such as by a CLIP displacement assay of the invention or by an APIPS assay. In some embodiments, the MHCII loading assay of the invention is carried out on a peptide identified as having high affinity for MHCII by a competition assay of the invention.

In some embodiments, a peptide is tested first by a CLIP displacement assay of the invention, then by an APIPS assay, then by a competition assay of the invention, and then by the MHCII loading assay of the invention.

### Testing tolerogenicity - ex vivo tolerance assay

In another aspect the invention provides an *ex vivo* method for testing the ability of a peptide to induce tolerance of an antigen comprising providing T cells from an animal that has been injected with the peptide, stimulating the T cells with the antigen, and measuring T cell activation by measuring BrdU incorporation, EdU incorporation, Ki67 levels, IL2 secretion and/or IFNy secretion. This method is referred to herein as an "ex *vivo* tolerance assay".

BrdU (5-bromo-2'-deoxyuridine) and EdU (5-ethynyl-2'-deoxyuridine) are synthetic nucleoside analogues of thymidine. BrdU or EdU incorporated into newly synthesized DNA of replicating cells can be detected by antibodies specific for BrdU or EdU, and thereby used as markers for proliferating cells. In some embodiments T cell activation is measured by measuring BrdU incorporation. In some embodiments T cell activation is measured by measuring EdU incorporation.

Ki-67 is a nuclear protein involved in transcription of ribosomal RNA. Ki-67 is present in the cell during the cell cycle but is absent from non-dividing cells. Ki-67 can therefore be used as a marker for proliferating cells. Staining with an antibody against Ki-67 may be used to measure cell proliferation. In some embodiments T cell activation is measured by measuring Ki67 levels.

In some embodiments T cell activation is measured by measuring IL2 secretion.

In some embodiments T cell activation is measured by additionally measuring IFNy secretion.

In some embodiments the animal has been immunised with the antigen. In some embodiments the animal is immunised with the antigen about 10-20 days after the animal is first injected with the peptide. In some embodiments the animal is immunised with the antigen about 15 days after the animal is first injected with the peptide. In some embodiments T cells are stimulated with the antigen about 10 days after the animal is immunised with the antigen. In some embodiments the T cells are stimulated with the antigen for about 48-96 hours. In some embodiments the T cells are stimulated with the antigen for about 72 hours. In some embodiments the peptide has been injected using a dose escalation schedule.

In some embodiments the animal is a mouse. In some embodiments the mouse is a HLA-DR2tg or a HLA-DR3tg mouse. In some embodiments the T cells are in a sample of lymph node cells and splenocytes.

In some embodiments the animal is a human. In some embodiments the T cells are in a sample of peripheral blood mononuclear cells (PBMCs).

In some embodiments the *ex vivo* tolerance assay of the invention may be combined with one or more of the other methods described herein for testing peptides for properties associated with tolerogenicity. Thus in some embodiments the invention provides a method for identifying a tolerogenic peptide comprising the *ex vivo* tolerance assay of the invention. In some embodiments the invention provides a method for identifying a tolerogenic peptide comprising providing T cells from an animal that has been injected with the peptide, stimulating the T cells with the antigen, and measuring T cell activation by measuring BrdU incorporation, EdU incorporation, Ki67 levels and/or IL2 secretion, wherein the method further comprises a method for identifying a peptide comprising a T cell epitope, and/or a method for testing the ability of a peptide to bind to MHCII without having undergone processing, and/or a method for testing the binding affinity of the peptide for MHCII, and/or a method for testing the *in vivo* solubility of the peptide, and/or a method for determining the effect of the peptide on cytokine secretion.

In some embodiments, the *ex vivo* tolerance assay of the invention is carried out on a peptide identified as comprising a T cell epitope, such as by a CLIP displacement assay of the invention. In some embodiments, the *ex vivo* tolerance assay of the invention is carried out on a peptide identified as being able to bind MCHII without having undergone processing, such as by a CLIP displacement assay of the invention or by an APIPS assay. In some embodiments, the *ex vivo* tolerance assay of the invention is carried out on a peptide identified as having high affinity for MHCII by a competition assay of the invention. In some embodiments, the *ex vivo* tolerance assay of the invention is carried out on a peptide identified as having good *in vivo* solubility by an MHCII loading assay of the invention.

In some embodiments, a peptide is tested first by a CLIP displacement assay of the invention, then by an APIPS assay, then by a competition assay of the invention, then by an MHCII loading assay of the invention, and then by an *ex vivo* tolerance assay of the invention.

### Biomarker assay

In another aspect the invention provides a method for determining the effect of a peptide on cytokine secretion comprising measuring serum cytokine levels of a mouse that has been injected with the peptide.

The pattern of cytokines secreted in response to the peptide demonstrates that the peptide binds MHCII and activates T cells *in vivo.* The change in the cytokine secretion pattern after repeated peptide injections, such as increased secretion of IL10, indicates tolerance induction.

In some embodiments the mouse is immunised with the peptide about 15-25 days before being injected with the peptide.

In some embodiments the mouse is immunised with the peptide about 21 days before being injected with the peptide.

In some embodiments the mouse is injected with about 100 µg of the peptide.

In some embodiments the mouse is a HLA-DRtg mouse.

In some embodiments cytokine levels are measured using
electrochemiluminescence.

In some embodiments IL2 levels are measured.

In some embodiments interleukin 6 (IL6) levels are measured.

In some embodiments interleukin 10 (IL10) levels are measured.

In some embodiments interleukin 12/interleukin 23p40 (IL12/IL23p40) levels are measured.

In some embodiments interleukin 17 (IL17) levels are measured.

In some embodiments IFNy levels are measured.

In some embodiments the biomarker assay of the invention may be combined with one or more of the other methods described herein for testing peptides for properties associated with tolerogenicity. Thus in some embodiments the invention provides a method for identifying a tolerogenic peptide comprising the biomarker assay of the invention. In some embodiments the invention provides a method for identifying a tolerogenic peptide comprising determining the effect of a peptide on cytokine secretion comprising measuring serum cytokine levels of a mouse that has been injected with the peptide, wherein the method further comprises a method for identifying a peptide comprising a T cell epitope, and/or a method for testing the ability of a peptide to bind to MHCII without having undergone processing, and/or a method for testing the binding affinity of the peptide for MHCII, and/or a method for testing the *in vivo* solubility of the peptide, and/or a method for testing the ability of a peptide to induce tolerance of an antigen.

In some embodiments, the biomarker assay of the invention is carried out on a peptide identified as comprising a T cell epitope, such as by a CLIP displacement assay of the invention. In some embodiments, the biomarker assay of the invention is carried out on a peptide identified as being able to bind MCHII without having undergone processing, such as by a CLIP displacement assay of the invention or by an APIPS assay. In some embodiments, the biomarker assay of the invention is carried out on a peptide identified as having high affinity for MHCII by a competition assay of the invention. In some embodiments, the biomarker assay of the invention is carried out on a peptide identified as having good *in vivo* solubility by an MHCII loading assay of the invention.

In some embodiments, a peptide is tested first by a CLIP displacement assay of the invention, then by an APIPS assay, then by a competition assay of the invention, then by an MHCII loading assay of the invention, then by an *ex vivo* tolerance assay of the invention, and then by a biomarker assay of the invention.

### Methods for identifying tolerogenic peptides

In another aspect the invention provides a method for selecting or identifying a tolerogenic peptide comprising any of the methods of the invention.

The methods of the invention described herein may be used in isolation, or in combination with some or all of the other methods of the invention in a method for identifying tolerogenic peptides.

The invention provides a method for identifying a tolerogenic peptide comprising
identifying a peptide comprising a T cell epitope using a method according to the invention, and/or
testing the ability of the peptide to bind to MHCII without having undergone processing using a method according to the invention, and/or
testing the binding affinity of the peptide for MHCII using a method according to the invention, and/or
testing the *in vivo* solubility of the peptide using a method according to the invention, and/or
testing the ability of a peptide to induce tolerance of an antigen using a method according to the invention, and/or
determining the effect of the peptide on cytokine secretion using a method according to the invention.

In some embodiments the invention provides a method for identifying a tolerogenic peptide comprising
identifying a peptide comprising a T cell epitope using a CLIP displacement assay according to the invention, and/or
testing the ability of the peptide to bind to MHCII without having undergone processing using a CLIP displacement assay according to the invention, and/or
testing the ability of the peptide to bind to MHCII without having undergone processing using an APIPS assay according to the invention, and/or
testing the binding affinity of the peptide for MHCII using a competition assay according to the invention, and/or
testing the *in vivo* solubility of the peptide using an MHCII loading assay according to the invention, and/or
testing the ability of a peptide to induce tolerance of an antigen using an *ex vivo* tolerance assay according to the invention, and/or
determining the effect of the peptide on cytokine secretion using a biomarker assay according to the invention.

In some embodiments the invention provides a method for identifying a tolerogenic peptide comprising
a method for identifying a peptide comprising a T cell epitope and/or for testing the ability of a peptide to bind to MHCII without having undergone processing comprising adding a peptide to MHCII-CLIP, adding T cells specific for an antigen, and measuring T cell activation, and/or
a method for testing the ability of the peptide to bind to MHCII without having undergone processing by treating an antigen processing independent presentation system (APIPS) with the peptide, adding T cells specific for the peptide to the APIPS, and measuring T cell activation, and/or
a method for testing the binding affinity of a peptide for MHCII comprising adding a test peptide and a control peptide to MHCII, adding T cells specific for the control peptide, and measuring T cell activation, and/or
a method for testing the *in vivo* solubility of a peptide comprising co-culturing dendritic cells from a mouse that has been injected with the peptide with T cells specific for the peptide, and measuring T cell activation, wherein the T cells are primary T cells, T cell clones or T cell hybridomas, and/or
an *ex vivo* method for testing the ability of a peptide to induce tolerance of an antigen comprising providing T cells from an animal that has been injected with the peptide, stimulating the T cells with the antigen, and measuring T cell activation by measuring BrdU incorporation, EdU incorporation, Ki67 levels, IL2 secretion and/or IFNy secretion, and/or
a method for determining the effect of a peptide on cytokine secretion comprising measuring serum cytokine levels of a mouse that has been injected with the peptide.

In some embodiments the method for identifying tolerogenic peptides comprises one method selected from the following: the CLIP displacement assay, the APIPS assay, the competition assay, the MHCII loading assay, the *ex vivo* tolerance assay, the biomarker assay. In some embodiments the method comprises two assays selected therefrom. In some embodiments the method comprises three assays selected therefrom. In some embodiments the method comprises four assays selected therefrom. In some embodiments the method comprises five assays selected therefrom. In some embodiments the method comprises all six assays listed.

In some embodiments, a given peptide may be tested in a first method selected from the methods described herein, then tested in a second method selected from the methods described herein, then tested in a third method selected from the methods described herein, then tested in a fourth method selected from the methods described herein, then tested in a fifth method selected from the methods described herein, then tested in a sixth method selected from the methods described herein.

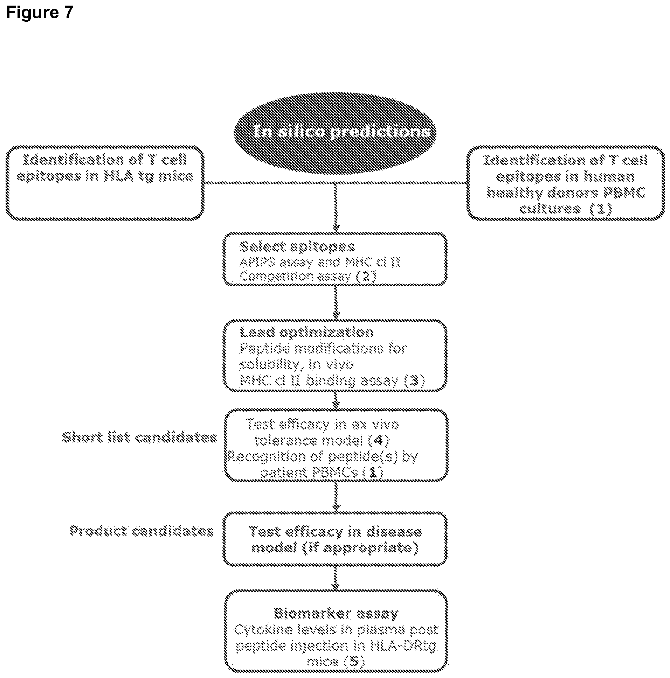

A schematic overview of how the methods of the invention may fit in an overarching method for identifying tolerogenic peptides is presented in **Fig. 7****.** T cell epitopes can be predicted *in silico* and identified in HLA transgenic mice and/or human primary blood mononuclear cell cultures. From these identified epitopes, apitopes can be selected using the CLIP displacement assay of the invention and/or an APIPS assay. The binding affinity of the apitopes for MHCII can be tested using the competition assay of the invention. After the apitopes have been screened in this manner they may be further modified to improve their physical properties, e.g. solubility and tested for the ability to bind MHC class II using the MHCII loading assay of the invention. Those peptides that display the ability to bind MHCII on dendritic cells may be further tested for their ability to induce tolerance in an *ex vivo* tolerance model and in a disease model if appropriate. These therapeutic candidates can also be tested in the biomarker assay of the invention. In this manner, a workflow for identifying potential therapeutic peptides for the treatment of autoimmune diseases is established.

The methods of the invention are not limited to particular combinations or a particular order. Preferably, the CLIP displacement assay is carried out first. In a preferred embodiment, the methods of the invention are carried out in the following order: CLIP displacement assay, APIPS assay, competition assay, MHCII loading assay, ex vivo tolerance assay, biomarker assay.

The invention provides a method for identifying a tolerogenic peptide comprising
identifying a peptide comprising a T cell epitope,
testing the ability of the peptide to bind to MHCII without having undergone processing,
testing the binding affinity of the peptide for MHCII,
testing the *in vivo* solubility of the peptide, and
testing the ability of a peptide to induce tolerance of an antigen.

### Peptides selected by the methods of the invention and compositions thereof

In another aspect the invention provides a tolerogenic peptide identified by a method of the invention.

In another aspect the invention provides a composition comprising a tolerogenic peptide identified by a method of the invention. In some embodiments the composition comprises more than one tolerogenic peptide identified by a method of the invention, such as at two or more such peptides, such as three or more such peptides. The peptides may be derivable from the same or different target antigen(s).

The composition may be in the form of a kit, in which some or each of the apitopes are provided separately for simultaneous, separate or sequential administration.

Alternatively (or in addition) if the composition (or any part thereof) is to be administered in multiple doses, each dose may be packaged separately.

The composition may comprise a therapeutically or prophylactically effective amount of the or each peptide and optionally a pharmaceutically acceptable carrier, diluent or excipient.

Also, in the compositions of the present invention, the or each peptide may be admixed with any suitable binder(s), lubricant(s), suspending agent(s), coating agent(s), or solubilising agent(s).

### Methods of treatment

In another aspect the invention provides a method for treating and/or preventing a disease in a subject comprising the step of administering a tolerogenic peptide identified by a method of the invention to the subject.

In another aspect the invention provides a method for treating and/or preventing a disease in a subject comprising the step of administering a composition comprising a tolerogenic peptide identified by a method of the invention to the subject.

In some embodiments the method comprises the following steps:
(i) identifying an antigen for the disease
(ii) identifying a tolerogenic peptide for the antigen; and
(iii) administering the tolerogenic peptide to the subject.

In another aspect the invention provides a tolerogenic peptide identified by a method of the invention for use in treating and/or preventing a disease.

In another aspect the invention provides a composition comprising a tolerogenic peptide identified by a method of the invention for use in treating and/or preventing a disease.

In another aspect the invention provides a use of tolerogenic peptide identified by a method of the invention in manufacture of a medicament for treatment and/or prevention of a disease.

In another aspect the invention provides a use of composition comprising a tolerogenic peptide identified by a method of the invention in manufacture of a medicament for treatment and/or prevention of a disease.

### Diseases

In some embodiments the disease is a hypersensitivity disorder. In some embodiments the disease is an autoimmune disease. In some embodiments the disease is an allergy. In some embodiments the disease is graft rejection.

An apitope for MHC class II is likely to be particularly useful in diseases which are mediated by CD4+ T cell responses. For example, diseases which are established or maintained by an inappropriate or excessive CD4+T cell response.

Such a peptide is likely to be particularly useful in the treatment of hypersensitivity disorders. Hypersensitivity reactions include:
(i) allergies, resulting from inappropriate responses to innocuous foreign substances;
(ii) autoimmune diseases, resulting from responses to self tissue antigens; and
(iii) graft rejection, resulting from responses to a transplant.

Examples of allergies include, but are not limited to: hay fever, extrinsic asthma, insect bite and sting allergies, food and drug allergies, allergic rhinitis, bronchial asthma chronic bronchitis, anaphylactic syndrome, urticaria, angioedema, atopic dermatitis, allergic contact dermatitis, erythema nodosum, erythema multiforme, Stevens-Johnson Syndrome, rhinoconjunctivitis, conjunctivitis, cutaneous necrotizing venulitis, inflammatory lung disease and bullous skin diseases.

Examples of the autoimmune diseases include, but are not limited to: rheumatoid arthritis (RA), myasthenia gravis (MG), multiple sclerosis (MS), systemic lupus erythematosus (SLE), autoimmune thyroiditis (Hashimoto's thyroiditis), Graves' disease, inflammatory bowel disease, autoimmune uveoretinitis, polymyositis and certain types of diabetes, systemic vasculitis, polymyositis-dermatomyositis, systemic sclerosis (scleroderma), Sjogren's Syndrome, ankylosing spondylitis and related spondyloarthropathies, rheumatic fever, hypersensitivity pneumonitis, allergic bronchopulmonary aspergillosis, inorganic dust pneumoconioses, sarcoidosis, autoimmune hemolytic anemia, immunological platelet disorders, cryopathies such as cryofibrinogenemia and autoimmune polyendocrinopathies.

A variety of tissues are commonly transplanted in clinical medicine, including kidney, liver, heart lung, skin, cornea and bone marrow. All grafts except corneal and some bone marrow grafts usually require long-term immunosuppression at present.

### Administration

In some embodiments the peptide or composition is administered subcutaneously.

In some embodiments the peptide or composition is administered intradermally.

In some embodiments the peptide is administered by a mucosal route.

In some embodiments the peptide is administered intranasally.

In some embodiments the peptide is administered in soluble form in the absence of adjuvant.

Studies have shown that peptide, when given in soluble form intraperitoneally (i.p.), intravenously (i.v.) or intranasally (i.n.) or orally can induce T cell tolerance (Anderton and Wraith (1998) as above; Liu and Wraith (1995) as above; Metzler and Wraith (1999) Immunology 97:257-263).

Studies in mice have demonstrated that the duration of peptide administration required to induce tolerance depends on the precursor frequency of T cells in the recipient (Burkhart et al (1999) as above). In many experimental studies, it has been shown that repeated doses of peptide are required to induce tolerance (Burkhart et al (1999) as above). The exact dose and number of doses of peptide will therefore depend on the individual, however, in a preferred embodiment a plurality of doses is administered.

If a plurality of peptides is administered simultaneously, they may be in the form of a "cocktail" which is suitable for administration in single or multiple doses. Alternatively it may be preferably to give multiple doses but vary the relative concentrations of the peptides between doses.

In some embodiments the peptide or composition is administered in multiple doses. In a preferred embodiment a "dose escalation" protocol may be followed, where a plurality of doses is given to the patient in ascending concentrations. Such an approach has been used, for example, for phospholipase A2 peptides in immunotherapeutic applications against bee venom allergy (Muller et al (1998) J. Allergy Clin Immunol. 101:747-754 and Akdis et al (1998) J. Clin. Invest. 102:98-106).

### EXAMPLES

### Materials and Methods

Materials and methods used in the following Examples are described here.

### Mice

DR3tg mice were bred under specific pathogen-free conditions externally at Charles River, UK, or at Innoser, Netherlands or Belgium. The DR3tg strain was originally created by Strauss et al (Strauss et al, Immunogenetics 1994). In brief, the genomic constructs used were a 6 kb *Nd*el fragment of a HLA-DRA genomic clone in pUC 13 and a 24 kb *C*/*a*I*xSa*/I fragment of cos 4.1, a cosmid (pTCF) containing the B gene of DRB1*0301. A solution containing 1-2 µg/mL of each construct was used for co-injection into fertilised eggs from (C57BL/6 × DBA/2) F1 donors mated with C57BL/6 males. The offspring has later been bred into the IA-beta knockout C57BL/6 genetic background (AB0 mice) lacking mouse MHC class II molecule expression. These DR3tg mice express the HLA-DRB1*0301 molecule but not the mouse MHC-II molecule. The mice were maintained by backcrossing to C57BL/6 and to B10.Q. Transgenic mice were identified by Southern blot analysis of tail DNA digested with *EcoR*I and probed with a 1.35 kb *BamH*I fragment of the DRA cDNA and a 1.25 kb *BamH*I fragment of the DRB1*0301 cDNA.

DR2tg mice were bred under specific pathogen-free conditions externally at Charles River, UK, or at Innoser, Netherlands or Belgium. HLA-DR2 transgenic (DR2tg) mice were originally obtained from Lars Fugger (Madsen et al., Nature Genetics 1999). In brief, DRα and DRβ chain cDNAs (DRA*0101 and DRB1*1501) were expressed by using the pDOI-5 expression vector which contains a mouse MHCII promotor. The constructs were injected into fertilised eggs from (DBA/2xC57BL/6)F1 matings. The mice were backcrossed into the IA-beta knockout C57BL/6 genetic background (AB0 mice) lacking mouse MHC class II molecule expression. The DR2tg mice express the HLA-DRB1*1501 molecule but not the mouse MHC molecule.

The DR4tg mouse strain was originally created by Lars Fugger et al. (Fugger et al, PNAS USA 1994). Endogenous MHC class II deletion was achieved by breeding onto the IA-b knockout (B6;129S2-H2dlAb1-Ea/J) mouse.

Animal studies were approved by the 'Ethical Committee for Animal experiments' (ECD) at Hasselt University and performed with the highest standards of care in a pathogen-free facility.

### Proteins and peptides

All single peptides were synthesized by Severn Biotech (Kidderminster, UK) and stored at a stock solution of 20 mg/ml in DMSO (Sigma-Aldrich) or of 4 mg/ml in phosphate-buffered saline (PBS) at -80°C. The peptides were synthesized with an N-terminal free amine and a C-terminal amide. Human SAg (S-arrestin) was produced in HEK293F cells (QBiologicals, Eurofins Amatsigroup, Ghent, Belgium). TSHR Human recombinant extracellular domain of TSHR (TSHR_{ECD}, AA19-417) was produced in a Trichoplusia ni larval expression system by using the Chesapeake PERL technology PERLXpress (Savage, MD, US).

Sequences of the peptides are presented in Table 1 below.

**Table 1**

| **Peptide** | **Sequence** | **SEQ ID No.** | **Antigen** |
|---|---|---|---|
| CLIP | PVSKMRMATPLLMQA | 1 | N/A |
| MBP83-99 | ENPVVHFFKNIVTPRTP | 2 | MBP |
| Peptide A | ISRIYVSIDVTLQQLESHSFYNLSKVTHI | 3 | TSHR |
| Peptide B | IYVSIDVTLQQLESH | 4 | TSHR |
| Peptide C | KKKYVSIDVTLQQLEKKK | 5 | TSHR |
| Peptide D | VIGLTFRRDLYFSRVQVYPPVG | 6 | S-Ag |
| Peptide E | TFRRDLYFSRVQ | 7 | S-Ag |
| Peptide F | MAASGKTSKSEPNHVIFKKISRDKSVTIYLGNRDYIDHVSQV | 8 | S-Ag |
| Peptide G | FKKISRDKSVTIY | 9 | S-Ag |
| Peptide H | KKGPRCLTRYYSSFVNMEGKK | 10 | FVIII |
| Peptide J | NPILLWQPIPVHTVPLSEDQ | 11 | PAP* |
| Peptide K | LTLLPLLANNRERRGIALDGKIKHEDTNLASSTIIKEG | 12 | S-Ag |
| Peptide L | GLKMFPDLTKVYSTD | 13 | TSHR |
| Peptide M | KKEIFKKISRDKSVTIYLEKK | 14 | S-Ag |
| Peptide N | GILVSYQIKVK | 15 | S-Ag |
| Peptide O | KKKGILVSYQIKVKKKK | 16 | S-Ag |
| Peptide P | KKIFKKISRDKSVTIYLKK | 17 | S-Ag |
| Peptide Q | KKKIFKKISRDKSVTIYLKKK | 18 | S-Ag |

| | | | |
|---|---|---|---|
| * Peptide J is a known HLA-DR2-binding peptide from Prostate Acid Phosphatase (PAP) described in Klyushnenkova et al. Prostate. 2007;67(10):1019-28.) | | | |

TSHR sequence (SEQ ID No. 19) - entry P16473 in UniProtKB database (https://www.uniprot.org/): 
MRPADLLQLVLLLDLPRDLGGMGCSSPPCECHQEEDFRVTCKDIQRIPSLPPSTQTLKLI
ETHLRTIPSHAFSNLPNISRIYVSIDVTLQQLESHSFYNLSKVTHIEIRNTRNLTYIDPD
ALKELPLLKFLGIFNTGLKMFPDLTKVYSTDIFFILEITDNPYMTSIPVNAFQGLCNETL
TLKLYNNGFTSVQGYAFNGTKLDAVYLNKNKYLTVIDKDAFGGVYSGPSLLDVSQTSVTA
LPSKGLEHLKELIARNTWTLKKLPLSLSFLHLTRADLSYPSHCCAFKNQKKIRGILESLM
CNESSMQSLRQRKSVNALNSPLHQEYEENLGDSIVGYKEKSKFQDTHNNAHYYVFFEEQE
DEIIGFGQELKNPQEETLQAFDSHYDYTICGDSEDMVCTPKSDEFNPCEDIMGYKFLRIV
VWFVSLLALLGNVFVLLILLTSHYKLNVPRFLMCNLAFADFCMGMYLLLIASVDLYTHSE
YYNHAIDWQTGPGCNTAGFFTVFASELSVYTLTVITLERWYAITFAMRLDRKIRLRHACA
IMVGGWVCCFLLALLPLVGISSYAKVSICLPMDTETPLALAYIVFVLTLNIVAFVIVCCC
YVKIYITVRNPQYNPGDKDTKIAKRMAVLIFTDFICMAPISFYALSAILNKPLITVSNSK
ILLVLFYPLNSCANPFLYAIFTKAFQRDVFILLSKFGICKRQAQAYRGQRVPPKNSTDIQ
VQKVTHDMRQGLHNMEDVYELIENSHLTPKKQGQISEEYMQTVL
S-Ag sequence (SEQ ID No. 20) - entry P10523 in UniProtKB database (https://www.uniprot.org/):

### Set-up of T cell line (TCL)

HLA-DRtg mice were immunised subcutaneously in the base of the tail with 50 µg peptide emulsified in CFA (peptide/CFA). Ten days after immunisation, draining lymph nodes (LN) and spleens were harvested. LN cells and splenocytes were isolated and CD4⁺ T cells were isolated by negative selection using Magnisort Mouse CD4 Isolation kit (ThermoFisher Scientific) according to the manufacturer's instructions. Irradiated (3000 rad) splenocytes were used as antigen-presenting cells (APC). 5×10⁶ APC + 2.5×10⁶ CD4⁺ T cells were cultured in X-vivo 15 medium (supplemented with 2mM L-glutamine, 50 U/mL penicillin and 50 U/mL streptomycin; Lonza) in 6-well plates in the presence of 0,1; 1; 2,5 or 5 µg/ml of peptide. At day 4, 20U/ml of rIL-2 (R&D Systems, Abingdon, UK) was added. At day 7, TCL cells were counted and cultured with fresh APC, peptides and IL-2, all at the same concentrations as above. On day 10, 20 U/ml of rIL-2 was added. On day 14, TCL cultures were used as such, or CD4⁺ cells were selected.

### Example 1 - CLIP displacement assay

The ability of a known tolerogenic peptide MBP 83-99 to displace CLIP from MHCII was tested. MBP 83-99 is a dominant HLA-DR2 restricted epitope of MBP having the sequence ENPWHFFKNIVTPRTP (SEQ ID No. 2).

MHCII (HLA-DR2) with bound CLIP (MHCII-CLIP) was affixed to wells of a 96-well plate using neutravidin and biotin. In particular, 96-well plates were coated with 15 µg/ml neutravidin in PBS overnight at 4°C. Plates were washed twice with wash buffer (0.1% BSA/PBS) and blocked with 1% BSA in PBS for 2 hours at room temperature. Plates were washed twice with wash buffer and 0.125 µg/ml of biotin-MHCII-CLIP in PBS was added for a 2-hour incubation at room temperature in a humid atmosphere. Plates were washed 4 times with wash buffer, leaving wells coated in MHCII-CLIP. Positive control wells were coated with MHCII with bound MBP 83-99 (instead of CLIP).

The wells containing MHCII-CLIP were incubated with different concentrations of MBP 83-99 for 16 hours then washed.

A T cell line specific for MBP was added to the wells and incubated at 37°C for 24 hours.

T cell activation was assessed by measuring secreted IL2. The results are presented in Figure 1. When MBP 83-99 at 30 µM or 3 µM is added to MHCII-CLIP ("CLIP monomer") T cells are activated, indicating that at these concentrations MBP 83-99 can displace CLIP from MHCII.

### Example 2 - APIPS assay

APIPS assays were performed using three combinations of different types of APCs and T cells.

The types of APCs used were either primary splenocytes from HLA-DRtg mice or cell line APCs.

The types of T cells used were T cell hybridomas, T cell lines or primary T cells.

For each combination, 5×10⁴ T cells were cultured with peptide (10 or 25 µg/ml) and APCs (5×10⁴ splenocytes or 2.5×10⁴ cell line cells).

For each combination, fixed and unfixed ("fresh") APCs were used. To fix APCs, cells were incubated with 0.5% paraformaldehyde (Merck, Darmstadt, Germany) (pH7) at room temperature for 2 minutes (if the APCs were primary splenocytes) or 5 minutes (if the APCs were cell lines). The fixation reaction was stopped by adding 0.4M glycine (Sigma-Aldrich) and washing the cells in RPMI-10%FCS.

For each combination, reactivity of the T cells towards human S-Ag or TSHR (10 or 25 µg/ml) was measured to identify cryptic epitopes.

T cell activation was assessed by measuring cytokine levels using ELISA (R&D Systems, Abingdon, UK).

*A. APIPS* assay *using splenocyte APCs with T cell hybridomas* Antigen-specific T cell hybridoma clones were incubated for 48 hours with known tolerogenic TSHR peptides and splenocytes from HLA-DRtg mice (APCs).

T cell hybridoma activation was assessed by measuring IL2 production. The results are presented in Figure 2A. The data show that T cell hybridomas are activated in response to peptides presented by splenocyte APCs. T cell hybridomas and splenocytes are therefore an effective T cell-APC combination for use in APIPS assays.

### B. APIPS assay using cell line APCs with T cell lines

T cell lines were incubated for 24 hours with known tolerogenic S-Ag peptides and APC cell lines. The APC cell lines used were VAVY cells (HLA-DR3 expressing) or MGAR cells (HLA-DR2 expressing).

T cell activation was assessed by measuring IFNy production. The results are presented in Figure 2B. The data show that T cell lines are activated in response to peptides presented by APC cell lines. T cell lines and APC cell lines are therefore an effective T cell-APC combination for use in APIPS assays.

### C. APIPS assay using cell line APCs with primary T cells

Primary T cells were incubated for 24 hours with known tolerogenic S-Ag peptides and APC cell lines. The APC cell lines used were VAVY cells (HLA-DR3 expressing) or MGAR cells (HLA-DR2 expressing).

To generate primary peptide-specific primary T cells, HLA-DRtg mice were immunised subcutaneously in the base of the tail with 50 µg peptide emulsified in CFA (peptide/CFA). Ten days after immunisation, draining lymph nodes (LN) and spleens were harvested. LN cells and splenocytes were isolated and CD4⁺ T cells were isolated by negative selection using Magnisort Mouse CD4 Isolation kit (ThermoFisher Scientific) according to the manufacturer's instructions.

T cell activation was assessed by measuring IFNy production. The results are presented in Figure 2C. The data show that primary T cells are activated in response to peptides presented by APC cell lines. Primary T cells and APC cell lines are therefore an effective T cell-APC combination for use in APIPS assays.

### Example 3 - Competition assay

An *in vitro* competition assay for assessing the binding affinity of a test peptide for MHCII was tested using test peptides known to have good affinity for MHCII (peptides J and L) and poor affinity for MHCII (peptides K and M).

T cell hybridoma clones specific for a control peptide were incubated with fixed APCs and a test peptide. During the incubation the test peptide binds to MHCII on the APCs.

In particular, T cell hybridoma clones (5×10⁵ cells) were co-cultured with 5×10⁵ fixed APCs for 0.5 hour in a 96-well flat bottom plate in the presence of 50 µg/ml, 10 µg/ml and 1 µg/ml test peptide in RPMI-1640 medium (supplemented with 15% FBS, 2 mM L-glutamine, 1 mM sodium-pyruvate, 50 U/mL penicillin and 50 U/mL streptomycin; Lonza). To fix APCs, cells were incubated with 0.5% paraformaldehyde (Merck, Darmstadt, Germany) (pH7) for 5 min at room temperature (RT). The fixation reaction was stopped by adding 0.4M glycine (Sigma-Aldrich) and washing the cells in supplemented RPMI-1640 medium.

The APCs used were MGAR cells (HLA-DR2 expressing) and VAVY cells (HLA-DR3 expressing).

After the 0.5 hour incubation with test peptide, control peptide was added to the cultures at concentrations ranging between 0.0125 µg/ml and 0.2 µg/ml.

After 48 hours, the response of the T cell hybridomas was assessed by measuring secreted IL2. The results are shown in Figure 3. In particular, Figures 3A and 3B show data for HLA-DR2 expressing APCs (MGAR cells) and Figures 3C and 3D show data for HLA-DR3 expressing APCs (VAVY cells) respectively.

Peptide H (see x-axes of Figures 3A and 3B) is the control peptide for HLA-DR2 binding and has the sequence KKGPRCLTRYYSSFVNMEGKK (SEQ ID No. 10).

Peptide C (see x-axes of Figure 3C and 3D) is the control peptide for HLA-DR3 binding and has the sequence KKKYVSIDVTLQQLEKKK (SEQ ID No. 5).

The *in vitro* competition assay clearly indicates whether a peptide can bind MHCII.

In particular, when no test peptide is added, T cell activation levels increase with increasing control peptide concentration (see 0 µg/ml line in Figures 3A-3D). The control peptide can bind to MHCII without competition and thereby activate T cells in a concentration-dependent fashion.

With sufficient quantities of a test peptide that can bind to MHCII, increasing control peptide concentration has little effect on T cell activation (see 50 and 10 µg/ml lines in Figures 3A and 3C). The test peptide outcompetes the control peptide for MHCII binding therefore T cells are activated less.

In contrast, if the test peptide cannot bind to MHCII, increasing control peptide concentration increases T cell activation to the same degree as if no test peptide were present (see 50, 10 and 1 µg/ml lines in Figures 3B and 3D, which show no difference to the 0 µg/ml line). The test peptide does not compete with the control peptide for MHCII binding, so the control peptide can activate T cells in a concentration-dependent fashion.

### Example 4 - In vivo MHCII loading assay

An *in vivo* MHCII loading assay for assessing solubility of peptides was tested using a peptide known to have poor solubility (peptide O) and a peptide known to have good solubility (peptide N). The test peptides were injected into mice and the extent to which the peptides bound MHCII on dendritic cells was assessed.

In particular, HLA-DRtg mice were injected with 100 µg of peptide in 100 µl PBS subcutaneously in the flank. Control animals received a subcutaneous injection of 100 µl PBS. After 2 hours, spleens were harvested and single-cell suspensions were made. CD11c⁺ cells (splenic dendritic cells) were positively selected using CD11c microbeads according to the manufacturer's instructions (Miltenyi Biotec, Bergisch Gladbach, Germany). Average purities of >92% were reached.

1×10⁵ CD11c⁺ cells were co-cultured with 1×10⁵ CD4⁺ cells (primary T cells) in round bottom 96-well plates in X-vivo 15 medium (supplemented with 2mM L-glutamine, 50 U/mL penicillin and 50 U/mL streptomycin; Lonza). The CD4⁺ cells were isolated from HLA-DRtg mice that were immunised subcutaneously in the base of the tail with 50 µg peptide emulsified in CFA (peptide/CFA). Ten days after immunisation, draining lymph nodes (LN) and spleens were harvested. LN cells and splenocytes were isolated and CD4⁺ T cells were isolated by negative selection using Magnisort Mouse CD4 Isolation kit (ThermoFisher Scientific) according to the manufacturer's instructions.

After 72 hours, supernatant of the CD11c⁺-CD4⁺ co-cultures was collected. CD4⁺ T cell activation was analysed by IFNy ELISA (R&D Systems, Abingdon, UK). In a parallel experiment, CD4⁺ T cell responses towards peptide added *in vitro* were assessed to make sure the T cells recognize the peptides presented by the CD11c⁺ cells.

Results of the *in vivo* assay are presented in Figure 4. The insoluble peptide (peptide N) did not cause T cell activation, whereas the soluble peptide (peptide O) did, indicating that the assay can identify peptides of suitable solubility.

### Example 5 - Ex vivo tolerance assay

HLA-DRtg mice were injected subcutaneously in the flank region with 0.1 µg, 1 µg and 10 µg of peptide on days -15, -13 and -11 respectively, followed by 3 injections of 100 µg peptide on days -8, -6 and -4 (dose escalation schedule). On day 0, the mice were immunised subcutaneously in the base of the tail with 50 µg antigen (parental peptide) emulsified in CFA (peptide/CFA).

Ten days after immunisation, draining lymph nodes (LN) and spleens were harvested. LN cells and splenocytes were isolated and cultured in X-vivo 15 medium (supplemented with 2mM L-glutamine, 50 U/mL penicillin and 50 U/mL streptomycin; Lonza) in 96-well flat bottom plates.

To investigate antigen-induced cell proliferation, 0.5×10⁶ cells/well were cultured (200 µl/well) for 72 hours with different antigen concentrations (0-25 µg/ml) or with 12.5 µg/ml purified protein derivative (PPD; priming control; Statens serum institut, Copenhagen, Denmark). After 72 hours, supernatant was harvested and stored at - 80°C until further analysis.

IFNy concentrations in supernatants were assessed by cytokine ELISA (R&D Systems, Abingdon, UK) and cell activation was measured. The results are presented in Figure 5.

### Example 6 - Biomarker assay

To determine the effects of injection with a tolerogenic peptide on *in vivo* cytokine secretion, HLA-DRtg mice were immunised subcutaneously in the base of the tail with 50 µg peptide C (sequence KKKYVSIDVTLQQLEKKK, SEQ ID No. 5) emulsified in CFA (peptide/CFA).

21 days post-immunisation mice were injected with 100 µg of peptide or vehicle.

2 hours after injection, blood of these mice was collected and serum cytokine levels analysed using an electrochemiluminescent assay (U-plex assay; Meso Scale Diagnostics (MSD)) according to manufacturer's instructions.

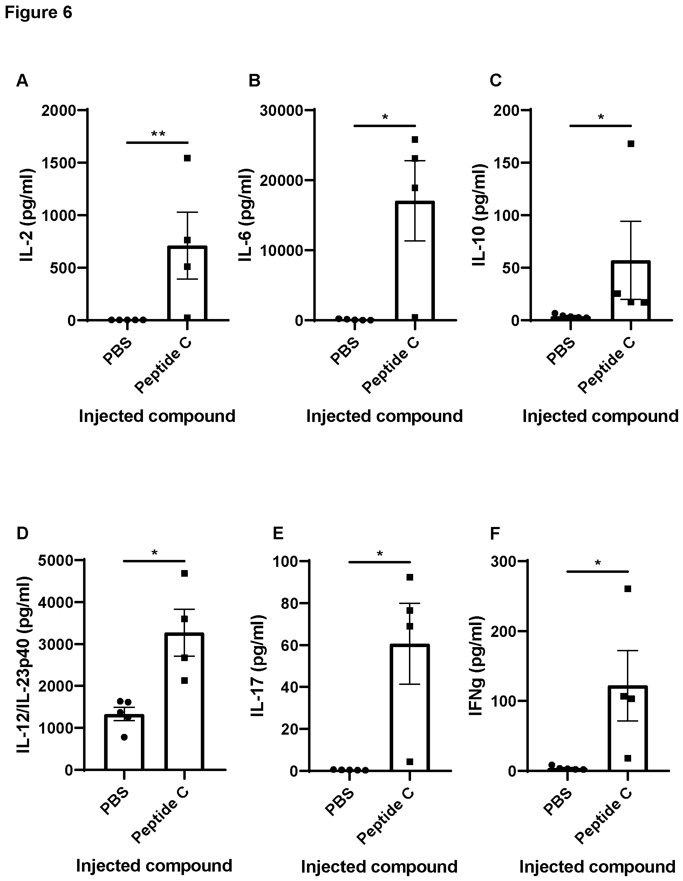

The results of the assay are presented in Figure 6.

The invention also relates to the following aspects as defined in the following numbered paragraphs:
1. A method for identifying a peptide comprising a T cell epitope, wherein said method comprises the steps of:
   (i) contacting a complex of major histocompatibility complex class II (MHCII) and Class II-associated invariant chain peptide (CLIP) (MHCII-CLIP) with a peptide,
   (ii) adding T cells specific for an antigen; and
   (iii) measuring activation of said T cells.
2. A method for testing the ability of a peptide to bind to MHCII without having undergone processing, wherein said method comprises the steps of:
   (i) contacting a complex of major histocompatibility complex class II (MHCII) and Class II-associated invariant chain peptide (CLIP) (MHCII-CLIP) with a peptide;
   (ii) adding T cells specific for an antigen; and
   (iii) measuring activation of said T cells.
3. A method according to paragraph 1 or 2 wherein the T cells are primary T cells, T cell clones or T cell hybridomas, or are from a T cell line.
4. A method according to paragraph 3 wherein the T cells are from a T cell line.
5. A method according to paragraph 3 wherein the T cells are from peripheral blood mononuclear cells (PBMCs) isolated from a subject.
6. A method according to any of paragraphs 1-5 wherein MHCII is Human Leukocyte Antigen - DR (HLA-DR) isotype 2 (HLA-DR2), HLA-DR3 or HLA-DR4.
7. A method according to paragraph 6 wherein MHCII is HLA-DR2.
8. A method according to any of paragraphs 1-7 wherein T cell activation is measured by measuring cytokine secretion and/or T cell proliferation.
9. A method according to paragraph 8 wherein T cell activation is measured by measuring interleukin 2 (IL2) secretion and/or interferon gamma (IFNγ) secretion.
10. A method according to paragraph 9 wherein T cell activation is measured by measuring IL2 secretion.
11. A method according to any of paragraphs 8-10 wherein T cell activation is measured by measuring ³H-thymidine incorporation, BrdU incorporation, EdU incorporation or Ki67 levels.
12. A method according to any of paragraphs 1-11 wherein CLIP comprises the sequence PVSKMRMATPLLMQA (SEQ ID No. 1).
13. The method according to any of paragraphs 1-12 wherein the method is an *in vitro* method.
14. A method according to paragraph 13 wherein MHCII-CLIP is affixed to a culture plate.
15. A method according to any of paragraphs 1-14 wherein MHCII-CLIP comprises biotin.
16. A method according to paragraph 15 wherein MHCII-CLIP is affixed to a culture plate by biotin-neutravidin.
17. Use of CLIP in the method according to any of paragraphs 1-16.
18. A use according to paragraph 17 wherein CLIP comprises the sequence PVSKMRMATPLLMQA (SEQ ID No. 1).
19. A method for testing the binding affinity of a peptide for MHCII, wherein said method comprises the steps of:
   (i) adding a test peptide to MHCII,
   (ii) adding a control peptide
   (iii) adding T cells specific for the control peptide; and
   (iii) measuring T cell activation.
20. A method according to paragraph 19 wherein the MHCII is on antigen-presenting cells (APCs).
21. A method according to paragraph 20 wherein the APCs are fixed.
22. A method according to paragraph 20 wherein the APCs are fresh.
23. A method according to any of paragraphs 19-22 wherein the APCs are primary APCs or from an APC line.
24. A method according to paragraph 23 wherein the APCs are MGAR cells.
25. A method according to paragraph 23 wherein the APCs are VAVY cells.
26. A method according to paragraph 23 wherein the APCs are BM14 cells.
27. A method according to any of paragraphs 19-26 wherein MHCII is HLA-DR2, HLA-DR3 or HLA-DR4.
28. A method according to paragraph 27 wherein MHCII is HLA-DR2.
29. A method according to paragraph 28 wherein the control peptide consists of the sequence KKGPRCLTRYYSSFVNMEGKK (SEQ ID No. 10).
30. A method according to paragraph 27 wherein MHCII is HLA-DR3 or HLA-DR4.
31. A method according to paragraph 30 wherein the control peptide consists of the sequence KKKYVSIDVTLQQLEKKK (SEQ ID No. 5).
32. A method according to any of paragraphs 19-31 wherein the T cells are primary T cells, T cell clones or T cell hybridomas, or are from a T cell line.
33. A method according to paragraph 32 wherein the T cells are T cell hybridomas.
34. A method according to any of paragraphs 19-33 wherein T cell activation is measured by measuring cytokine secretion and/or T cell proliferation.
35. A method according to paragraph 34 wherein T cell activation is measured by measuring IL2 secretion and/or IFNy secretion.
36. A method according to paragraph 35 wherein T cell activation is measured by measuring IL2 secretion.
37. A method according to any of paragraphs 33-36 wherein T cell activation is measured by measuring ³H-thymidine incorporation, BrdU incorporation, EdU incorporation or Ki67 levels.
38. A method according to any of paragraphs 19-37 wherein the test peptide is added to MHCII before the control peptide.
39. A method according to paragraph 38 wherein the test peptide is incubated with MHCII for about 15 to 45 minutes before adding the control peptide.
40. A method according to any of paragraphs 19-39 wherein the method is an *in vitro* method.
41. Use of a peptide consisting of the sequence KKGPRCLTRYYSSFVNMEGKK (SEQ ID No. 10) in the method according to any of paragraphs 19-40.
42. Use of a peptide consisting of the sequence KKKYVSIDVTLQQLEKKK (SEQ ID No. 5) in the method according to any of paragraphs 19-40.
43. A method for testing the *in vivo* solubility of a peptide, said method comprising the steps of:
   (i) providing dendritic cells from a mouse that has been injected with the peptide;
   (ii) co-culturing said dendritic cells with T cells specific for said peptide; and
   (iii) measuring activation of said T cells.
44. A method according to paragraph 43 wherein the T cells are primary T cells, T cell clones or T cell hybridomas.
45. A method according to paragraph 44 wherein the T cells are primary T cells.
46. A method according to any of paragraph 43-44 wherein the mouse is a HLA-DR2 transgenic (HLA-DR2tg) or HLA-DR3 transgenic (HLA-DR3tg) mouse.
47. A method according to any of paragraphs 43-46 wherein T cell activation is measured by measuring cytokine secretion and/or T cell proliferation.
48. A method according to paragraph 47 wherein T cell activation is measured by measuring IL2 secretion and/or IFNy secretion.
49. A method according to paragraph 48 wherein T cell activation is measured by measuring IL2 secretion.
50. A method according to paragraph 48 wherein T cell activation is measured by measuring IFNy secretion.
51. A method according to any of paragraphs 47-50 wherein T cell activation is measured by measuring ³H-thymidine incorporation, BrdU incorporation, EdU incorporation or Ki67 levels.
52. A method according to any of paragraphs 43-51 wherein the mouse has been injected with about 100 µg of peptide.
53. A method according to any of paragraphs 43-52 wherein the mouse has been injected subcutaneously.
54. A method according to any of paragraphs 43-54 wherein the dendritic cells are harvested from the spleen of the mouse.
55. An *ex vivo* method for testing the ability of a peptide to induce tolerance to an antigen, said method comprising the steps of:
   (i) providing T cells from an animal that has been injected with the peptide;
   (ii) stimulating said T cells with the antigen; and
   (iii) measuring activation of said T cells.
56. A method according to paragraph 55 wherein T cell activation is measured by measuring BrdU incorporation, EdU incorporation, Ki67 levels, IL2 secretion and/or IGNy secretion.
57. A method according to paragraph 56 wherein T cell activation is measured by measuring Ki67 levels.
58. A method according to paragraph 56 wherein T cell activation is measured by measuring IFNγ secretion.
59. A method according to any of paragraphs 55-58 wherein the animal has been immunised with the antigen.
60. A method according to paragraph 59 wherein the animal is immunised with the antigen about 10-20 days after the animal is first injected with the peptide.
61. A method according to paragraph 60 wherein the animal is immunised with the antigen about 15 days after the animal is first injected with the peptide.
62. A method according to any of paragraphs 59-61 wherein T cells are stimulated with the antigen about 10 days after the animal is immunised with the antigen.
63. A method according to any of paragraphs 55-62 wherein the T cells are stimulated with the antigen for about 48-96 hours.
64. A method according to paragraph 63 wherein the T cells are stimulated with the antigen for about 72 hours.
65. A method according to any of paragraphs 55-64 wherein the peptide has been injected using a dose escalation schedule.
66. A method according to any of paragraphs 55-65 wherein the animal is a mouse.
67. A method according to paragraph 66 wherein the mouse is a HLA-DR2tg, a HLA-DR3tg or a HLA-DR4tg mouse.
68. A method according to paragraph 66 or 67 wherein the T cells are in a sample of lymph node cells and splenocytes.
69. A method according to any of paragraphs 55-68 wherein the animal is a human.
70. A method according to paragraph 69 wherein the T cells are in a sample of peripheral blood mononuclear cells (PBMCs).
71. A method for determining the effect of a peptide on cytokine secretion comprising measuring serum cytokine levels of a mouse that has been injected with the peptide.
72. A method according to paragraph 71 wherein the mouse is immunised with the peptide about 15-25 days before being injected with the peptide.
73. A method according to paragraph 72 wherein the mouse is immunised with the peptide about 21 days before being injected with the peptide.
74. A method according to any of paragraphs 71-73 wherein the mouse is injected with about 100 µg of the peptide.
75. A method according to any of paragraphs 71-74 wherein the mouse is a HLA-DRtg mouse.
76. A method according to any of paragraphs 71-75 wherein cytokine levels are measured using electrochemiluminescence.
77. A method according to any of paragraphs 71-76 wherein IL2 levels are measured.
78. A method according to any of paragraphs 71-77 wherein interleukin 6 (IL6) levels are measured.
79. A method according to any of paragraphs 71-78 wherein interleukin 10 (IL10) levels are measured.
80. A method according to any of paragraphs 71-79 wherein interleukin 12/interleukin 23p40 (IL12/IL23p40) levels are measured.
81. A method according to any of paragraphs 71-80 wherein interleukin 17 (IL17) levels are measured.
82. A method according to any of paragraphs 71-81 wherein IFNγ levels are measured.
83. A method for identifying a tolerogenic peptide comprising the steps of:
   (i) performing a CLIP displacement assay according to any of paragraphs 1-16, and/or
   (ii) performing an *in vitro* competition assay according to any of paragraphs 19-40, and/or
   (iii) performing an MHCII loading assay according to any of paragraphs 43-54, and/or
   (iv) performing a CLIP displacement assay with patient PBMCs according to any of paragraphs 55-70, and/or
   (v) testing the efficacy of the peptide in a disease model; and/or
   (vi) performing a biomarker assay according to any of paragraphs 71-82.
84. A method according to paragraph 83 further comprising testing the ability of the peptide to bind to MHCII without having undergone processing by
   treating an antigen processing independent presentation system (APIPS) with the peptide,
   adding T cells specific for the peptide to the APIPS, and
   measuring T cell activation.
85. A method according to paragraph 84, wherein the APIPS comprises:
   (i) fixed APCs
   (ii) lipid membranes comprising MHCII; or
   (iii) plate-bound MHCII.
86. A method according to paragraphs 85 wherein the APCs are primary APCs or from an APC line.
87. A method according to paragraph 86 wherein the primary APCs are splenocytes.
88. A method according to paragraph 86 wherein the APCs are MGAR cells.
89. A method according to paragraph 86 wherein the APCs are VAVY cells.
90. A method according to any of paragraphs 84-89 wherein the T cells added to the APIPS are primary T cells, T cell clones or T cell hybridomas, or are from a T cell line.
91. A method according to paragraph 90 wherein the T cells are primary T cells.
92. A method according to any of paragraphs 84-91 wherein T cell activation is measured by measuring cytokine secretion and/or T cell proliferation.
93. A method according to paragraph 92 wherein T cell activation is measured by measuring IL2 secretion and/or IFNγ secretion.
94. A method according to paragraph 93 wherein T cell activation is measured by measuring IFNγ secretion.
95. A method according to any of paragraphs 92-94 wherein T cell activation is measured by measuring ³H-thymidine incorporation, BrdU incorporation, EdU incorporation or Ki67 levels.
96. A method for identifying a tolerogenic peptide comprising
   identifying a peptide comprising a T cell epitope,
   testing the ability of the peptide to bind to MHCII without having undergone processing,
   testing the binding affinity of the peptide for MHCII,
   testing the *in vivo* solubility of the peptide, and
   testing the ability of a peptide to induce tolerance of an antigen.
97. A tolerogenic peptide identified by a method according to any preceding paragraph.
98. A composition comprising a tolerogenic peptide according to paragraph 97.
99. A method for treating and/or preventing a disease in a subject comprising the step of administering a peptide according to paragraph 97 or a composition according paragraph 98 to the subject.
100. A method according to paragraph 99 comprising the following steps:
   (i) identifying an antigen for the disease
   (ii) identifying a tolerogenic peptide for the antigen; and
   (iii) administering the tolerogenic peptide to the subject.
101. A method according to paragraph 99 or 100 wherein the peptide or composition is administered in multiple doses.
102. A method according to any of paragraphs 99-101, wherein the peptide or composition is administered subcutaneously.
103. A method according to any of paragraphs 99-101, wherein the peptide or composition is administered intradermally.
104. A tolerogenic peptide according to paragraph 97 or a composition according to paragraph 98 for use in treating and/or preventing a disease.
105. Use of tolerogenic peptide according to paragraph 97 or a composition according to paragraph 98 in manufacture of a medicament for treatment and/or prevention of a disease.

## Claims

1. A method for testing the binding affinity of a peptide for MHCII, wherein said method comprises the steps of:
(i) adding a test peptide to MHCII,
(ii) adding a control peptide
(iii) adding T cells specific for the control peptide; and
(iii) measuring T cell activation.

2. A method according to claim 1 wherein the MHCII is on antigen-presenting cells (APCs), preferably wherein the APCs are
(a) fixed or fresh; and/or
(b) primary APCs or from an APC line; and/or
(c) MGAR cells, VAVY cells or BM14 cells.

3. A method according to claim 1 or claim 2 wherein MHCII is HLA-DR2, HLA-DR3 or HLA-DR4.

4. A method according to any one of claims 1 to 3 wherein
(a) MHCII is HLA-DR2, and the control peptide consists of the sequence KKGPRCLTRYYSSFVNMEGKK (SEQ ID No. 10); or
(b) MHCII is HLA-DR3 or HLA-DR4, and the control peptide consists of the sequence KKKYVSIDVTLQQLEKKK (SEQ ID No. 5).

5. A method according to any one of claims 1 to 4 wherein the T cells are primary T cells, T cell clones or T cell hybridomas, or are from a T cell line, preferably wherein the T cells are T cell hybridomas.

6. A method according to any one of claims 1 to 5 wherein T cell activation is measured by measuring cytokine secretion and/or T cell proliferation, preferably wherein T cell activation is measured by measuring
(a) IL2 secretion and/or IFNγ secretion, more preferably IL2 secretion; and/or
(b) ³H-thymidine incorporation, BrdU incorporation, EdU incorporation or Ki67 levels.

7. A method according to any one of claims 1 to 6 wherein the test peptide is added to MHCII before the control peptide, preferably wherein the test peptide is incubated with MHCII for about 15 to 45 minutes before adding the control peptide.

8. A method according to any one of claims 1 to 7 wherein the method is an *in vitro* method.

9. Use of a peptide consisting of the sequence KKGPRCLTRYYSSFVNMEGKK (SEQ ID No. 10) in the method according to any one of claims 1 to 8.

10. Use of a peptide consisting of the sequence KKKYVSIDVTLQQLEKKK (SEQ ID No. 5) in a method according to any one of claims 1 to 8.

11. A tolerogenic peptide identified by a method according to any one of claims 1 to 8.

12. A composition comprising a tolerogenic peptide according to claim 11.

13. A tolerogenic peptide according to claim 11 or a composition according to claim 12 for use in a method for treating and/or preventing a disease in a subject, wherein the method comprises the step of administering the peptide or the composition to the subject.

14. The tolerogenic peptide or composition for use according to claim 13 wherein the method comprises the following steps:
(i) identifying an antigen for the disease
(ii) identifying a tolerogenic peptide for the antigen; and
(iii) administering the tolerogenic peptide to the subject.

15. The tolerogenic peptide or composition for use according to claim 13 or claim 14 wherein
(a) the peptide or composition is administered in multiple doses; and/or
(b) the peptide or composition is administered subcutaneously or intradermally.
